# EUROPEAN PATENT APPLICATION

(11) **EP 1 325 912 A1**
(43) Date of publication of application: **09.07.2003**
(21) Application number: 01974820.1
(22) Date of filing: 12.10.2001
(51) Int. Cl.: C07D 211/14, C07D 221/22, C07D 211/18, C07D 211/70, C07D 213/74, C07D 215/38, C07D 215/42, C07D 217/22, C07D 239/42, C07D 241/20, C07D 243/08, C07D 277/42, C07D 295/14, C07D 401/04, C07D 403/04, C07D 417/04, A61P 29/00, A61P 25/02

(54) **2,2-DIPHENYLBUTANAMIDE DERIVATIVES AND MEDICINES CONTAINING THE SAME**

(30) Priority: 12.10.2000 JP 2000311926; 27.12.2000 JP 2000398474
(71) Applicant: SSP Co., Ltd., Chuo-ku, Tokyo 103-8481 (JP)
(72) Inventor: OKADA, Tomomi, NARITA-SHI, Chiba 286-0013 (JP); KOMOTO, Teruo, CHIBA-SHI, Chiba 263-0002 (JP); SATO, Susumu, NARITA-SHI, Chiba 286-0011 (JP); OKA, Tetsuo, HIRATSUKA-SHI, Kanagawa 254-0806 (JP); SAKAMOTO, Takao, SENDAI-SHI, Miyagi 981-3212 (JP); KANAMARU, Yoshihiko, YACHIYO-SHI, Chiba 276-0033 (JP); MOGI, Kinichi, NARITA-SHI, Chiba 286-0036 (JP); MORIMOTO, Shinichi, NARITA-SHI, Chiba 286-0033 (JP); UMEHARA, Norimitsu, TOKOROZAWA-SHI, Saitama 359-0025 (JP); KAMEI, Junzo, YOKOHAMA-SHI, Kanagawa 236-0012 (JP)
(74) Representative: Wächtershäuser, Günter, Prof. Dr.
(86) International application number: JP0108982
(87) International publication number: WO02030896

(57) **Abstract**

2,2-Diphenylbutanamide derivatives represented by the following formula (1): 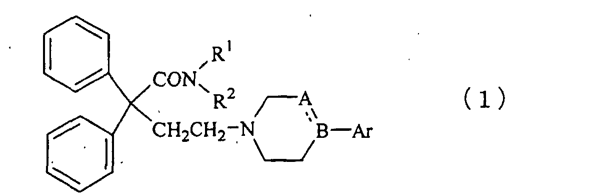 [wherein A represents -(CH₂)ₙ- (n is 1 or 2) or a methine (CH) group; when A is -CH₂-, B represents a methine group or a nitrogen atom, with A and B forming a single bond; when A is -(CH₂)₂-, B represents a nitrogen atom, with A and B forming a single bond; when A is a methine group, B represents a quaternary carbon atom, with A and B forming a double bond; each of R¹ and R², which are identical to or different from each other, represents a hydrogen atom, a lower alkyl group, or a cycloalkyl group, or R¹ and R² may form a heterocyclic ring together with the adjacent nitrogen atom; and Ar represents an optionally substituted phenyl group, bicyclic aromatic ring, monocyclic heterocyclic ring, bicyclic heterocyclic ring, or fluorene group]; or salts of the derivatives.

The derivatives or salts thereof exhibit excellent µ-opioid agonist activity and analgesic activity against neuropathic pain, and are useful as medicines such as peripheral analgesic drugs and neuropathic pain relieving drugs.

## Description

### Technical Field

The present invention relates to 2,2-diphenylbutanamide derivatives or salts thereof exhibiting excellent peripheral analgesic activity and excellent analgesic activity against neuropathic pains; and to medicines containing the derivatives or the salts.

### Background Art

Known analgesic drugs include central acting opioid analgesic drugs, such as morphine; non-steroidal anti-inflammatory drugs (NSAIDs) such as indomethacin; and local anesthetic drugs such as lidocaine (The Journal of Medicinal Chemistry, Vol. 42, No. 9, p. 1481, 1999, and references quoted therein).

However, morphine exhibits central acting adverse side effects, and thus a limitation is imposed on use of morphine. Meanwhile, existing non-steroidal anti-inflammatory drugs and local anesthetic drugs exhibit insufficient analgesic activity against some pains. Therefore, demand has arisen for drugs exhibiting safety and high analgesic activity as compared with the aforementioned drugs.

The presence of µ-receptors in peripheral organs has been reported in recent years, and analgesic activity expressed via the receptors is now being elucidated (The Journal of Pharmacology and Experimental Therapeutics, Vol. 248, No. 3, p. 1269, 1989; The Journal of Investigative Dermatology, Vol. 111, p. 297, 1988; and Drug Therapy, Vol. 323, p. 1685, 1995).

Japanese Patent Application Laid-Open (*kokai*) No. 47-173 discloses diarylpiperidinobutyramide compounds. Of the compounds, loperamide, which was developed as an antidiarrheal drug, is now under development as a peripheral analgesic drug (Anesthesiology, Vol. 90, p. 225, 1999; and The Journal of Pharmacology and Experimental Therapeutics, Vol. 289, p. 494, 1999).

However, loperamide does not necessarily exhibit satisfactory peripheral analgesic activity.

Neuropathic pains are caused by primary damage to the nervous system or functional disorder of the system, or induced by such damage or disorder. Examples of neuropathic pains include herpes zoster pains, diabetic pains, postoperative or post-traumatic chronic pains, pains after dental treatment, and pains after spinal cord injury/cerebral apoplexy. The level of neuropathic pain is considerably higher than that to be expected from the noxious stimulus applied to a part of the body suffering from neuropathic pain.

The aforementioned analgesic drugs exhibit low analgesic activity against neuropathic pains. For example, NSAIDs, which block generation of pain-producing substances, cannot relieve acute pains.

Therefore, an object of the present invention is to provide a compound exhibiting excellent peripheral analgesic activity and excellent analgesic activity against neuropathic pains.

### Disclosure of the Invention

In view of the foregoing, the present inventors have performed extensive studies in order to produce the aforementioned compound exhibiting excellent analgesic activity, and have found that 2,2-diphenylbutanamide derivatives represented by the following formula (1) exhibit remarkably excellent peripheral analgesic activity and neuropathic pain relieving activity as compared with the aforementioned loperamide, and are useful as medicines. The present invention has been accomplished on the basis of this finding.

Accordingly, the present invention provides a 2,2-diphenylbutanamide derivative represented by the following formula (1): 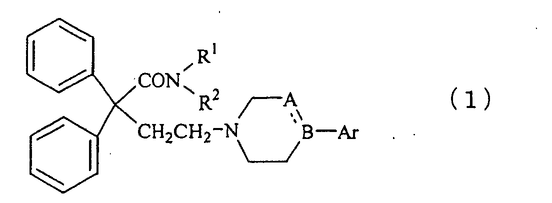 [wherein A represents -(CH₂)ₙ- (n is 1 or 2) or a methine (CH) group; when A is -CH₂-, B represents a methine group or a nitrogen atom, with A and B forming a single bond; when A is -(CH₂)₂-, B represents a nitrogen atom, with A and B forming a single bond; and when A is a methine group, B represents a quaternary carbon atom, with A and B forming a double bond;
each of R¹ and R², which are identical to or different from each other, represents a hydrogen atom, a lower alkyl group, or a cycloalkyl group; or R¹ and R² may form a heterocyclic ring together with the adjacent nitrogen atom; and Ar represents a phenyl group, a bicyclic aromatic ring, a monocyclic heterocyclic ring, a bicyclic heterocyclic ring, or a fluorene group, which may have a substituent represented by the following group: 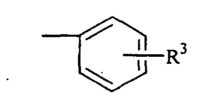 (wherein R³ represents a hydrogen atom, a halogen atom, a phenyl group, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; Y represents -COOR⁷, -OR⁸, -OCOR⁹, or-CONR¹⁰R¹¹ (wherein each of R⁷, R⁸, and R⁹ represents a hydrogen atom, a lower alkyl group, or a cycloalkyl group; and each of R¹⁰ and R¹¹ represents a hydrogen atom, a lower alkyl group, or a cycloalkyl group, or R¹⁰ and R¹¹ may form a heterocyclic ring together with the adjacent nitrogen atom); and m is 1 to 6)))]; or a salt of the derivative.

The present invention also provides a medicine comprising, as an active ingredient, the 2,2-diphenylbutanamide derivative (1) or a salt thereof.

The present invention also provides a medicinal composition comprising the 2,2-diphenylbutanamide derivative (1) or a salt thereof and a pharmacologically acceptable carrier.

The present invention also provides a method for treating peripheral pains or neuropathic pains, comprising administration of the 2,2-diphenylbutanamide derivative (1) or a salt thereof.

The present invention also provides use of the 2,2-diphenylbutanamide derivative (1) or a salt thereof for producing a peripheral analgesic drug or a neuropathic pain relieving drug.

### Brief Description of the Drawing

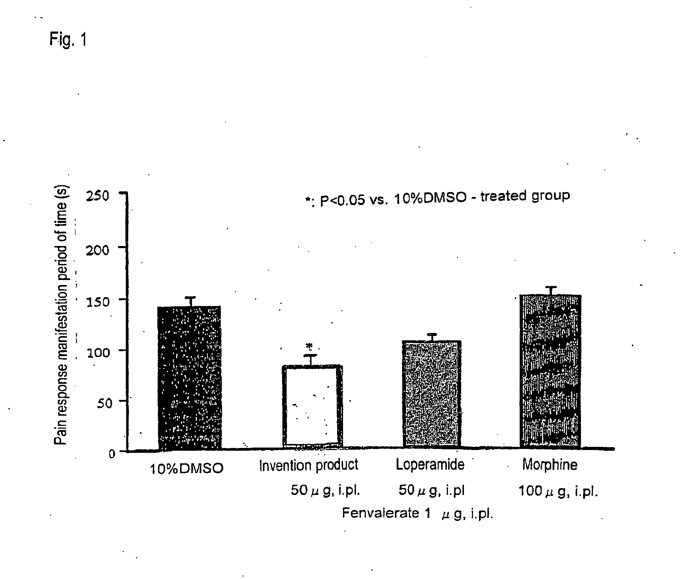

Fig. 1 shows pain response manifestation period of time as determined for respective drugs employed in Test Example 2.

### Best Mode for Carrying Out the Invention

In the 2,2-diphenylbutanamide derivatives (1) of the present invention, when n of A shown in formula (1) is 1, the ring assumes a piperazine ring. When n is 2, the ring assumes a homopiperazine ring. Of these, a piperazine ring is preferred (n = 1).

Examples of the lower alkyl group represented by R¹ and R² include C1-C6 linear or branched alkyl groups. Specific examples include a methyl group, an ethyl group, an n-propyl group, an i-propyl group, an n-butyl group, an i-butyl group, a sec-butyl group, a tert-butyl group, an n-pentyl group, and an n-hexyl group. Preferred examples of the cycloalkyl group include C3-C8 cycloalkyl groups. Specific examples include a cyclopropyl group, a cyclobutyl group, a cyclopentyl group, a cyclohexyl group, a cycloheptyl group, and a cyclooctyl group. Of these, R¹ and R² are particularly preferably a hydrogen atom and a C1-C6 alkyl group, respectively.

The number of atoms constituting the heterocyclic ring which is formed by R¹ and R² together with the adjacent nitrogen atom is preferably 3 to 6. Examples of the heterocyclic ring include an aziridine ring, a pyrrolidine ring, a piperidine ring, a piperazine ring, and a morpholine ring. A pyrrolidine ring is particularly preferred.

Examples of the group represented by Ar include bicyclic aromatic rings such as a naphthalene ring; monocyclic heterocyclic rings such as a pyridine ring, a pyrimidine ring, a pyrazine ring, and a thiazole ring; and bicyclic heterocyclic rings such as a quinoline ring and an isoquinoline ring.

Ar is preferably a phenyl group which may have a substituent. Examples of the halogen atom represented by R³ include a fluorine atom and a chlorine atom. Examples of the lower alkyl group include C1-C6 linear or branched alkyl groups. Specific examples include alkyl groups as described above.

R⁴ of the substituent -O-R⁴ represents a hydrogen atom, a lower alkyl group, or -(CR⁵R⁶)ₘ-Y. Examples of the lower alkyl group represented by R⁵ or R⁶ include C1-C6 linear or branched alkyl groups. Specific examples include a methyl group, an ethyl group, an n-propyl group, an i-propyl group, an n-butyl group, an i-butyl group, a sec-butyl group, a tert-butyl group, an n-pentyl group, and an n-hexyl group. Each of R⁵ and R⁶ is preferably a hydrogen atom. In -(CR⁵R⁶)ₘ-Y, m is 1 to 6, preferably 1 to 3.

In -COOR⁷, -OR⁸, -OCOR⁹, or -CONR¹⁰R¹¹ represented by Y, the lower alkyl group presented by R⁷, R⁸, R⁹, R¹⁰ or R¹¹ is a C1-C6 linear or branched alkyl group. Specific examples include a methyl group, an ethyl group, an n-propyl group, an i-propyl group, an n-butyl group, an i-butyl group, a sec-butyl group, a tert-butyl group, an n-pentyl group, and an n-hexyl group. Preferred examples of the cycloalkyl group include C3-C8 cycloalkyl groups. Specific examples include a cyclopropyl group, a cyclobutyl group, a cyclopentyl group, a cyclohexyl group, a cycloheptyl group, and a cyclooctyl group.

Preferably, R⁷ is a hydrogen atom or an ethyl group; R⁸ is a hydrogen atom or a methyl group; and R⁹ is a methyl group.

The number of atoms constituting the heterocyclic ring which is formed by R¹⁰ and R¹¹ together with the adjacent nitrogen atom is preferably 3 to 6. Examples of the heterocyclic ring include an aziridine ring, a pyrrolidine ring, a piperidine ring, a piperazine ring, and a morpholine ring.

Preferred examples of the phenyl group which may have a substituent include a phenyl group and a phenyl group having a substituent at the ortho position. Preferred examples of the substituent include a methyl group, a chlorine atom, a fluorine atom, a hydroxyl group, and a methoxy group.

No particular limitations are imposed on the salts of the 2,2-diphenylbutanamide derivatives of the present invention, so long as the salts are pharmacologically acceptable. Examples of the salts include addition salts of inorganic acids such as hydrochloric acid, sulfuric acid, hydrobromic acid, and phosphoric acid; and addition salts of organic acids such as formic acid, acetic acid, fumaric acid, maleic acid, and tartaric acid. The compounds of the present invention encompasses solvates such as hydrates, and polybasic acid salts.

The compounds of the present invention are represented by the following formulas (1-1), (1-2), and (1-3): 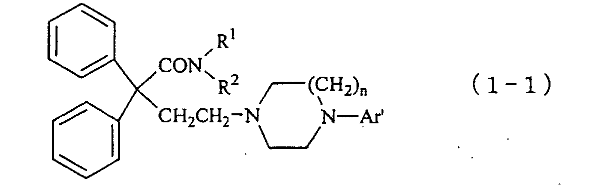 [wherein R¹, R², and n have the same meanings as described above; and Ar' represents a phenyl group, a bicyclic aromatic ring, a monocyclic heterocyclic ring, a bicyclic heterocyclic ring, or a fluorene group, which may have a substituent represented by the following group: 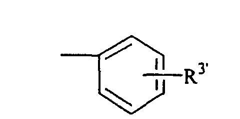 (wherein R^{3'} represents a hydrogen atom, a halogen atom, a phenyl group, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; and Y represents -COOR⁷, -OR⁸, or -OCOR⁹ (wherein each of R⁷, R⁸, and R⁹ represents a hydrogen atom, a lower alkyl group, or a cycloalkyl group), m is 1 to 6)))]; 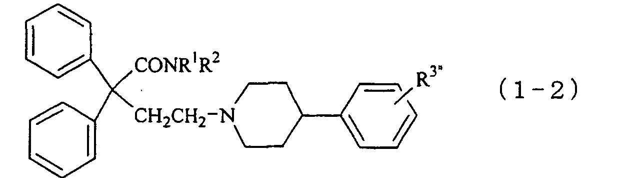 (wherein R¹ and R² have the same meanings as described above; and R³" represents a hydrogen atom, a halogen atom, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; and Y represents -COOR⁷, -OR⁸, -OCOR⁹, or -CONR¹⁰R¹¹ (wherein each of R⁷, R⁸, and R⁹ represents a hydrogen atom, a lower alkyl group, or a cycloalkyl group; and each of R¹⁰ and R¹¹ represents a hydrogen atom, a lower alkyl group, or a cycloalkyl group, or R¹⁰ and R¹¹ may form a heterocyclic ring together with the adjacent nitrogen atom) m is 1 to 6))); and 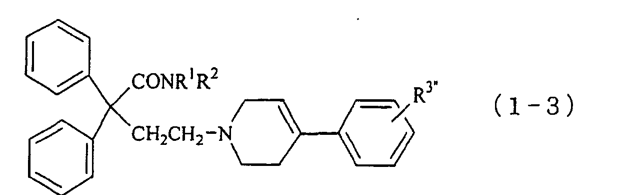 (wherein R¹, R², and R^{3"} have the same meanings as described above).

The 2,2-diphenylbutanamide derivative of the present invention and the salt thereof can be produced through, for example, the following Methods A through L.
Production of compounds represented by formula (1-1): 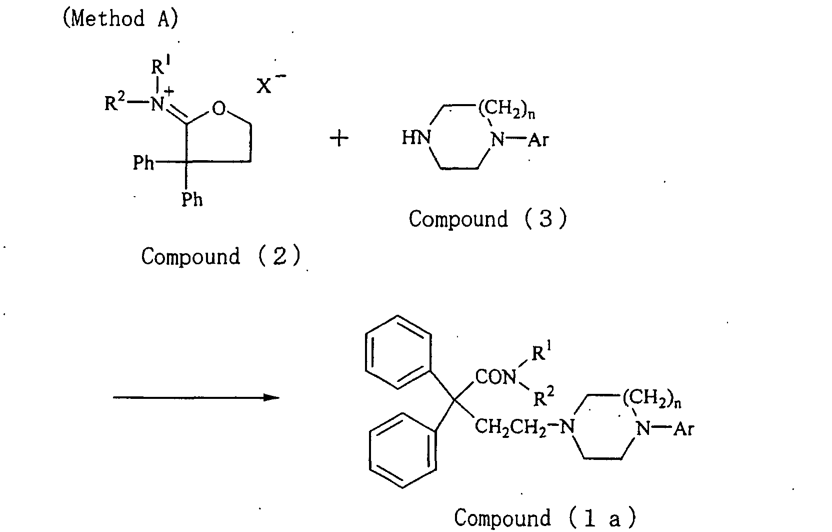 (In the above formula, X represents a halogen atom, and R¹, R², n, and Ar have the same meanings as described above.)

Specifically, furylideneammonium compound (2) is reacted with compound (3), to thereby yield compound (1a) of the present invention. This reaction is performed in the presence of a base (typically 2 to 5 equivalents, preferably 3 equivalents) at 40 to 140°C (preferably at 80 to 120°C) for 1 to 18 hours. This reaction may be performed in an anhydrous solvent such as benzene, toluene, xylene, tetrahydrofuran, or dimethylformamide. Examples of the base include inorganic bases such as sodium carbonate, potassium carbonate, sodium hydroxide, and potassium hydroxide; and organic bases such as triethylamine.

Compound (2) can be synthesized through a known method [R. A. Stokbrokx et al., J. Med. Chem., 16, 782 (1973)].

Compound (3) can be synthesized through the below-described method, or is commercially available.
1) Compound (3) represented by the following formula (3-1) is commercially available. 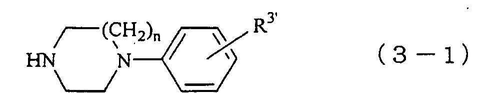 (In the above formula, n and R³' have the same meanings as described above.)
Examples of the commercially available compound include N-(2-methoxyphenyl)piperazine (n=1, R³=2-OCH₃).
2) The following compound (3-2) was synthesized through the below-described process. 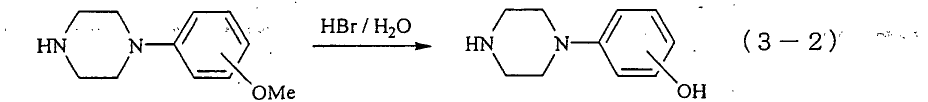
   Compound (3-2) may be synthesized under heating in hydrobromic acid. Alternatively, compound (3-2) may be produced through a synthesis method employing boron tribromide, or a method employing ethanethiol and aluminum chloride.
3) Active halides of the following heterocyclic compounds can be synthesized through nucleophilic substitution reaction between piperazine or homopiperazine and an active halide in a solvent under heating. 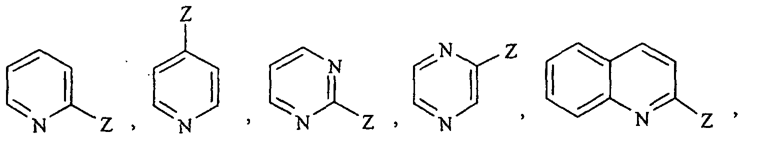 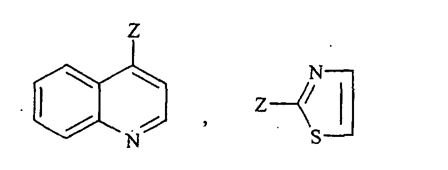 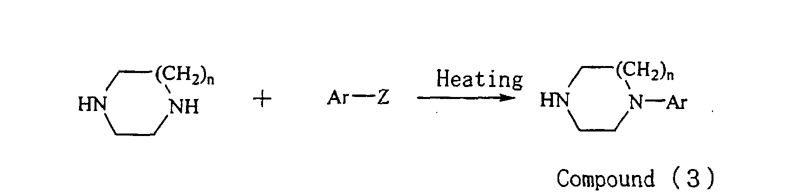 (In the above formula, Z represents a leaving group, and n and Ar have the same meanings as described above.)
   This reaction does not necessarily require a solvent. Specific examples of the solvent which may be employed include toluene, xylene, and pseudocumene. The reaction temperature is appropriately 100 to 170°C. The reaction time is preferably 4 to 24 hours.
   The following commercially available products may also be employed. 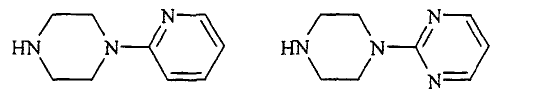
4) Compound (3) may be synthesized through methods described in the following publications.

1) S. L. Buchward et al., Angew. Chem. Int. Ed. Engl. 34, 1348 (1995).
2) J. P. Wolfe et al., Acc. Chem. Res., 31, 805-818(1998). 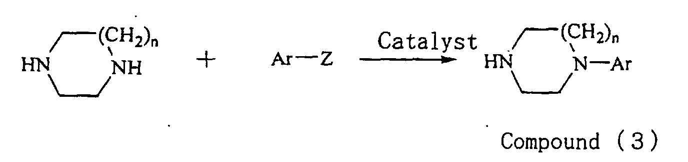 (In the above formula, n and Ar have the same meanings as described above.)
   Preferably, dichlorobis(tri-o-tolylphosphine)palladium(II) [PdCl₂(P(o-tolyl)₃)₂] is employed as a catalyst; a bromide of a halogen compound is employed as a leaving group; and potassium t-butoxide is employed as a base in an amount of 1 to 2 equivalents. Preferred examples of the solvent which is employed include toluene, xylene, and pseudocumene. The reaction temperature is appropriately 100 to 170°C. The amount of the catalyst is 0.5 to 10 mol%, preferably 5 mol%. The reaction time is preferably 4 to 24 hours. 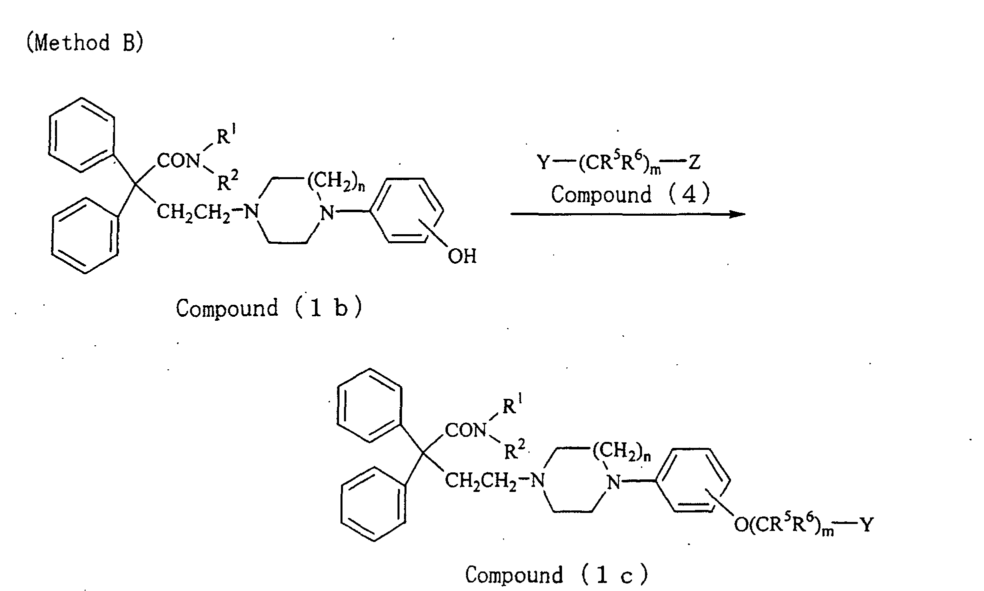
   Compound (1 c) (In the above formula, m is 1 to 6; R¹, R², n, R⁵, R⁶, and Y have the same meanings as described above; and Z represents a halogen atom, mesylate, tosylate, or triflate.)

Specifically, compound (1b) is reacted with compound (4), to thereby yield compound (1c) of the present invention. This reaction is performed in the presence of a base (typically 1 to 3 equivalents, preferably 1 to 1.5 equivalents) at 20 to 100°C (preferably at 20 to 60°C) for 2 to 36 hours. This reaction may be performed in an anhydrous solvent such as acetone, tetrahydrofuran, dimethylformamide, or dimethyl sulfoxide. Examples of the base include inorganic bases such as sodium carbonate, sodium hydroxide, and sodium hydride; and organic bases such as triethylamine. The base may be employed in combination with potassium iodide.

Compound (4) is readily available as a commercial reagent, or can be synthesized through a known method. 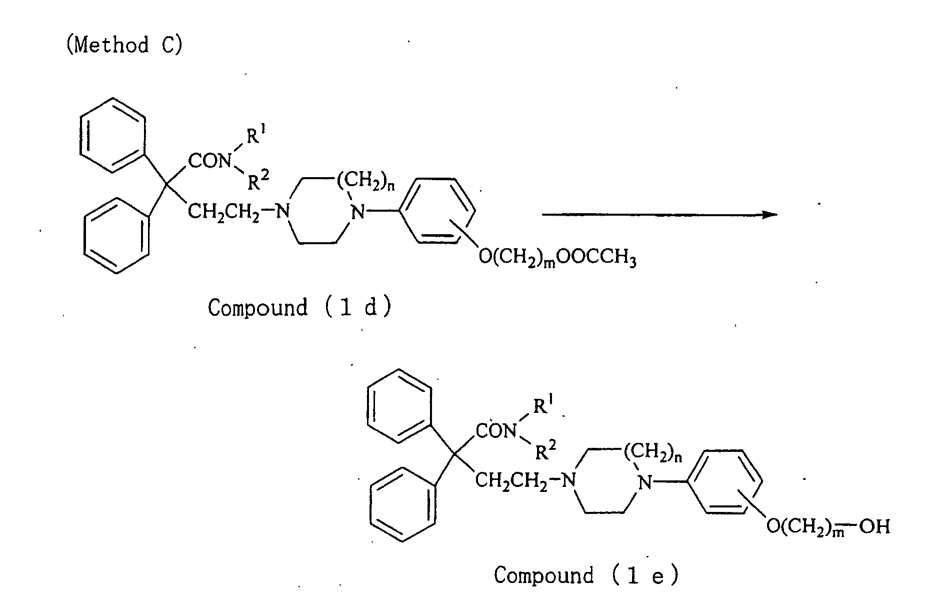 (In the above formula, R¹, R², n, and m have the same meanings as described above.)

Specifically, compound (1d) synthesized through method B (i.e., compound (1c) wherein R⁵ and R⁶ are H and Y is OAc) is hydrolyzed to thereby yield compound (1e) of the present invention. 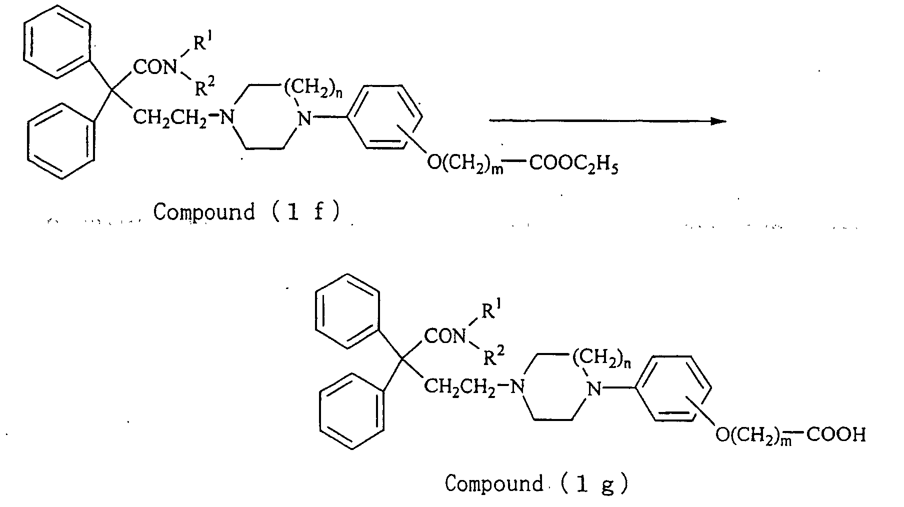 (In the above formula, R¹, R², n, and m have the same meanings as described above.)

Compound (1f) synthesized through method B (i.e., compound (1c) wherein R⁵ and R⁶ are H and Y is COOEt) is hydrolyzed to thereby yield compound (1g) of the present invention.

The aforementioned reactions are performed in the presence of a base (typically 1 to 3 equivalents, preferably 1 to 1.5 equivalents) at 20 to 40°C (preferably at 20 to 25°C) for 1 to 5 hours. The aforementioned reactions may be performed in a solvent which can be mixed with water, such as methanol, ethanol, dioxane, or tetrahydrofuran. Examples of the base include inorganic bases such as sodium hydroxide, potassium hydroxide, and sodium carbonate.
Production of compounds represented by formula (1-2) : 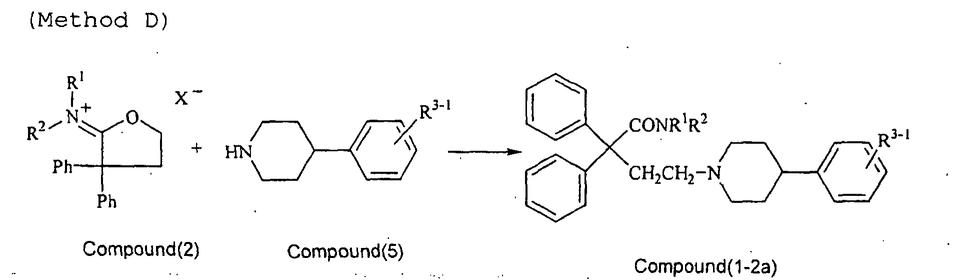 (In the above formula, X represents a halogen atom; R¹ and R² have the same meanings as described above; and R³⁻¹ represents a hydrogen atom, a fluorine atom, a chlorine atom, a hydroxy group, a lower alkyl group, or a lower alkoxy group.)

Specifically, furylideneammonium compound (2) is reacted with compound (5), to thereby yield compound (1-2a) of the present invention. This reaction is performed in the presence of a base (typically 2 to 5 equivalents, preferably 3 equivalents) at 40 to 100°C (preferably 50 to 60°C) for 1 to 18 hours. This reaction may be performed in an anhydrous solvent such as benzene, toluene, xylene, tetrahydrofuran, or dimethylformamide. Examples of the base include inorganic bases such as sodium carbonate, potassium carbonate, sodium hydroxide, and potassium hydroxide; and organic bases such as triethylamine. The aforementioned compound (2) can be synthesized through a known method [R. A. Stokbrokx et al., J. Med. Chem., 16, 782 (1973)]. The method for synthesizing the aforementioned compound (5) will be described below in Referential Example. 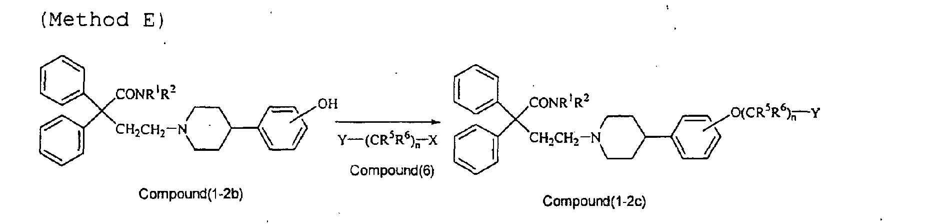 (In the above formula, R¹, R², R⁵, R⁶, n, and Y have the same meanings as described above; and X represents a halogen atom, mesylate, tosylate, or triflate.)

Specifically, compound (1-2b) is reacted with compound (6), to thereby yield compound (1-2c) of the present invention. This reaction is performed in the presence of a base (typically 1 to 3 equivalents, preferably 1 to 1.5 equivalents) at 20 to 100°C (preferably at 20 to 60°C) for 1 to 36 hours. This reaction may be performed in an anhydrous solvent such as acetone, tetrahydrofuran, dimethylformamide, or dimethyl sulfoxide. Examples of the base include inorganic bases such as sodium carbonate, sodium hydroxide, and sodium hydride; and organic bases such as triethylamine. The base may be employed in combination with potassium iodide. The aforementioned compound (6) is readily available as a commercial reagent, or can be synthesized through a known method. 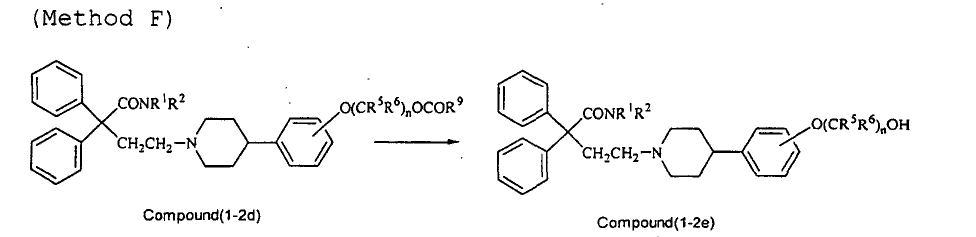 (In the above formula, R¹, R², R⁵, R⁶, R⁹, and n have the same meanings as described above.)

Compound (1-2e) of the present invention-can be produced through hydrolysis of compound (1-2d). Specifically, compound (1-2d) is hydrolyzed in the presence of a base (1 to 3 equivalents, preferably 1 to 1.5 equivalents) at 20 to 40°C (preferably at 20 to 25°C) for 1 to 5 hours, to thereby yield compound (1-2e) of the present invention. This reaction may be performed in a solvent which can be mixed with water, such as methanol, ethanol, dioxane, or tetrahydrofuran. Examples of the base include inorganic bases such as sodium hydroxide, potassium hydroxide, and sodium carbonate. 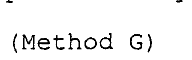 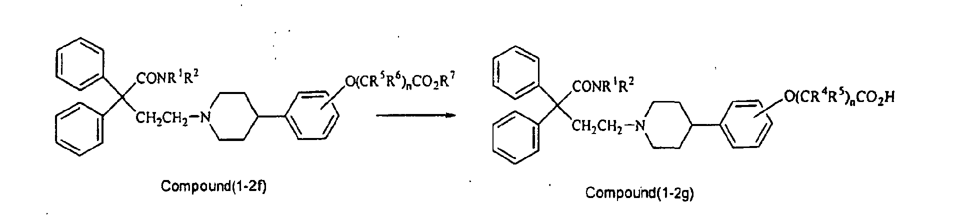 (In the above formula, R¹, R², R⁵, R⁶, R⁷, and n have the same meanings as described above.)

Specifically, compound (1-2f) is hydrolyzed to thereby yield compound (1-2g) of the present invention. This reaction is performed in the presence of a base (typically 1 to 3 equivalents, preferably 1 to 1.5 equivalents) at 20 to 40°C (preferably at 20 to 25°C) for 1 to 5 hours. This reaction may be performed in a solvent which can be mixed with water, such as methanol, ethanol, dioxane, or tetrahydrofuran. Examples of the base include inorganic bases such as sodium hydroxide, potassium hydroxide, and sodium carbonate.
Production of compounds represented by formula (1-3) : 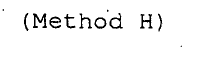 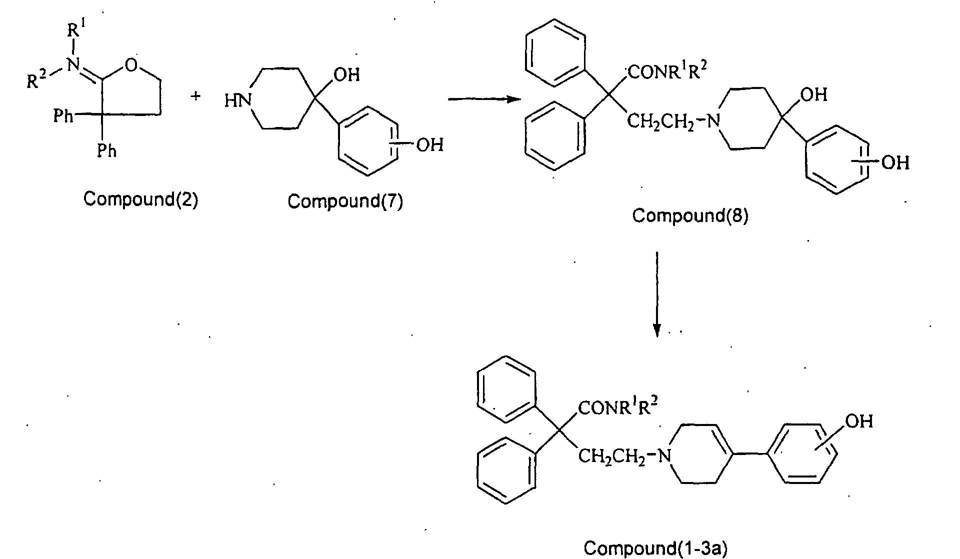 (In the above formula, R¹ and R² have the same meanings as described above.)

Compound (8) is produced from furylideneammonium compound (2) and compound (7) in a manner similar to that o (method A), and compound (8) is dehydrated, to thereby yiel compound (1-3a) of the present invention. The dehydration reaction is performed by adding excess acid to compound (8) and then refluxing the resultant mixture under heating for several hours. This reaction may be performed by use of a solvent such as alcohol, tetrahydrofuran, or dioxane. Examples of the acid which may be employed include hydrochloric acid and sulfuric acid.

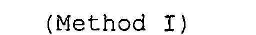

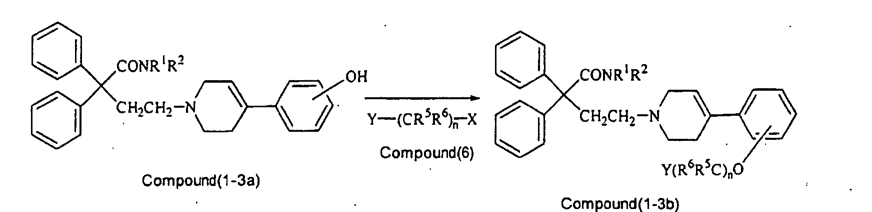

Compound(1-3b) (In the above formula, R¹, R², R⁵, R⁶, n, and Y have the same meanings as described above; and X represents a halogen atom, mesylate, tosylate, or triflate.)

The aforementioned reaction is performed in a manner similar to that of (method E). 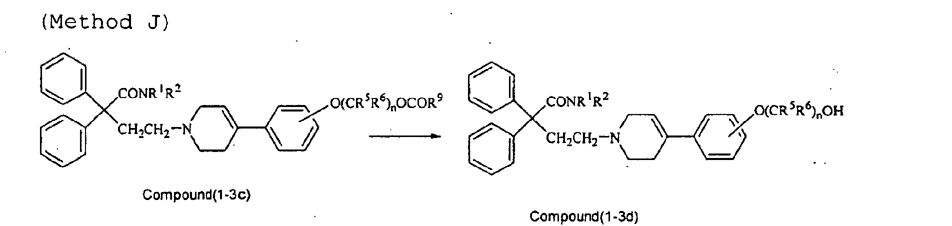 (In the above formula, R¹, R², R⁵, R⁶, R⁹, and n have the same meanings as described above.)

The aforementioned reaction is performed in a manner similar to that of (method F). Specifically, compound (1-3c) is hydrolyzed to thereby yield compound (1-3d) of the present invention. 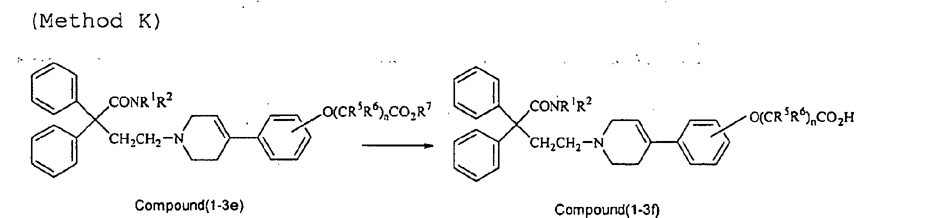 (In the above formula, R¹, R², R⁵, R⁶, R⁷, and n have the same meanings as described above.)

The aforementioned reaction is performed in a manner similar to that of (method G). Specifically, compound (1-3e) is hydrolyzed to thereby yield compound (1-3f) of the present invention. 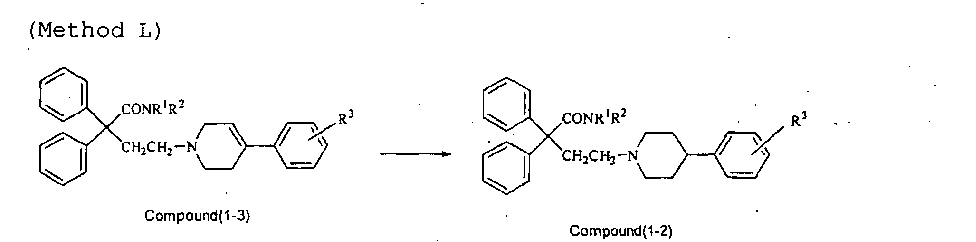 (In the above formula, R¹, R², and R³ have the same meanings as described above.)

Compound (1-3) is reduced to thereby yield compound (1-2) of the present invention. The reduction reaction is performed in a hydrogen atmosphere in the presence of a catalyst such as palladium, palladium-carbon, rhodium, or platinum black (1 to 50 wt.% on the basis of the entirety of compound (1-3)) at room temperature to an elevated temperature and under ambient pressure or with the application of pressure for 1 to 10 hours. Preferred solvents include ethanol, methanol, and water-containing alcohol. If desired, a small of amount of an acid may be added.

The salt of the 2,2-diphenylbutanamide derivative of the present invention is obtained through, for example, reaction between compound (1) and an acid (1 to 3 equivalents, preferably 1 equivalent) in an anhydrous solvent at 0 to 30°C for 0.1 to 0.5 hours. Preferred examples of the solvent include anhydrous ether, anhydrous tetrahydrofuran, anhydrous chloroform, anhydrous dioxane, and anhydrous acetone. Examples of the acid include the aforementioned acids.

The thus-obtained 2,2-diphenylbutanamide derivatives of the present invention or salts thereof may be purified by combination of typical techniques such as column chromatography and recrystallization.

As described below in Test Examples, the 2,2-diphenylbutanamide derivatives of the present invention or salts thereof exhibit excellent µ-opioid agonist activity and analgesic activity against pains induced by fenvalerate, and therefore are useful as medicines, such as neuropathic pain relieving drugs and peripheral analgesic drugs for mammals (including human) and other animals. The peripheral analgesic drug of the present invention can be employed for preventing or relieving pains caused by various diseases such as cancer and inflammatory diseases, surgical operation, injury, fracture, and burn.

Various physical forms of medicinal compositions can be produced from the 2,2-diphenylbutanamide derivatives of the present invention or salts thereof and pharmacologically acceptable carriers by means of a conventional method. No particular limitations are imposed on the manner of administration, which may be appropriately chosen in accordance with the purpose of treatment. For example, the composition may assume any physical form such as an oral drug, an injection, a suppository, an ointment, or a patch. Products of these physical forms can be produced through customary methods which have conventionally been known to persons skilled in the art.

An oral solid drug product is prepared as follows. The 2,2-diphenylbutanamide derivative of the present invention or a salt thereof is mixed with an excipient (and, if necessary, an additive such as a binder, a disintegrant, a lubricant, a coloring agent, a sweetening agent, or a flavoring agent), and the resultant mixture is processed through a routine method, to thereby produce a product such as tablets, coated tablets, granules, powder, or capsules.

An oral liquid drug product is prepared as follows. The 2,2-diphenylbutanamide derivative of the present invention or a salt thereof is mixed with, if necessary, an additive such as a sweetening agent, a buffer, a stabilizer, or a flavoring agent, and the resultant mixture is processed through a routine method, to thereby produce a product such as an internal liquid drug.

An injection is prepared as follows. The 2,2-diphenylbutanamide derivative of the present invention or a salt thereof is mixed with an additive such as a pH regulator, a buffer, a stabilizer, an isotonicity agent, or a local anesthetic, and the resultant mixture is processed through a routine method, to thereby produce an injection such as a subcutaneous injection, an intramuscular injection, or an intraveneous injection.

A suppository is prepared as follows. The 2,2-diphenylbutanamide derivative of the present invention or a salt thereof is mixed with a known carrier for producing drugs, such as polyethylene glycol, lanolin, cocoa butter, or fatty acid triglyceride (and, if necessary, an additive such as a surfactant), and the resultant mixture is processed through a routine method, to thereby produce a suppository.

An ointment is prepared as follows. The 2,2-diphenylbutanamide derivative of the present invention or a salt thereof is mixed with, if necessary, a generally employed base, stabilizer, humectant, or preservative, and the resultant mixture is processed through a routine method, to thereby produce an ointment.

A patch is produced by applying onto a conventional supporter the aforementioned ointment, cream, gel, or paste through a routine method.

The amount of the 2,2-diphenylbutanamide derivative of the present invention or a salt thereof to be incorporated into one physical form unit varies with the medical condition of the patient in need thereof or the physical form of the drug. Preferably, the amount of the derivative or the salt incorporated into one physical form unit is about 0.25 to about 100 mg in the case of -oral drugs, about 0.05 to about 20 mg in the case of injections, and about 0.1 to about 50 mg in the case of suppositories. The daily dose of a drug having the aforementioned physical form differs depending on the medical condition, body weight, age, and sex of the patient. The daily dose of the drug for an adult is usually about 0.005 to about 2 mg/kg, preferably about 0.01 to about 0.1 mg/kg. The drug is preferably administered once a day or about 2 to 4 times a day in a divided manner.

### Examples

The present invention will next be described in detail by way of examples, which should not be construed as limiting the invention.

### Referential Example 1

4-Bromo-2,2-diphenylbutyric acid (23 g, 72 mmol) was suspended in chloroform (150 mL). Thionyl chloride (20 mL, 270 mmol) was added dropwise at room temperature. Subsequently, DMF (0.2 mL) was added, and the mixture was heated and refluxed for 4 hours. Thereafter, the solvent was evaporated under reduced pressure, to thereby yield 23 g of 4-bromo-2,2-diphenylbutyric chloride (yield 94.7%).

Aqueous 50% dimethylamine solution (8 g, 90 mmol) and sodium carbonate (18 g, 170 mmol) were suspended in water (100 mL), and the suspension was cooled to a temperature between 0 and 5°C. To the cooled suspension was added dropwise the above-prepared 4-bromo-2,2-diphenylbutyric chloride (23 g, 68 mmol) dissolved in toluene (100 mL). The mixture was stirred for 2 hours, and thereafter, the aqueous layer was washed with toluene, followed by extraction with chloroform, washing with water, and drying. The solvent was evaporated under reduced pressure, and the residue was crystallized from methyl isobutyl ketone, to thereby yield 11 g of dimethyl(tetrahydro-3,3-diphenyl-2-furylidene)ammonium bromide (yield 46.8%).
¹H-NMR(CDCl₃)δ(ppm) : 2.96 (3H, s), 3.47 (2H, t), 3.83 (3H, s), 4.85 (2H, t),7.40-7.60 (10H, m)

### Referential Example 2

The procedure of Referential Example 1 was repeated except that pyrrolidine was used instead of aqueous 50% dimethylamine solution, whereby (tetrahydro-3,3-diphenyl-2-furylidene)pyrrolidinium bromide was obtained (yield 53.6%). ¹H-NMR(CDCl₃)δ(ppm) : 1.80-2.30 (4H, m), 2.88 (2H, t), 3.50 (2H, t), 4.37 (2H, t), 4.88 (2H, t), 7.25-7.70 (10H, m) Referential Example 3

Piperazine (610 mg, 7.08 mmol), 2-bromonaphthalene (1.04 g, 5.02 mmol), sodium t-butoxide (680 mg, 7.08 mmol), and dichlorobis(tri-o-tolylphosphine)palladium(II) [PdCl₂[P(o-tolyl)₃]₂] (200 mg, 0.247 mmol) were added to pseudocumene (bp 169°C; 20 mL), and the mixture was stirred under reflux for 24 hours. Subsequently, the reaction mixture was diluted by addition of tetrahydrofuran, and the diluted solution was filtered by use of Celite. The filtrate was concentrated under reduced pressure. The residue was subjected to silica gel-column chromatography, and the 7% MeOH/CHCl₃ eluate was concentrated under reduced pressure, to thereby yield 546 mg of the product of interest, 1-(2-naphthyl)piperazine, as a pale yellow solid (yield 51.5%). MS(EI) m/z : 212 (M⁺)
¹H-NMR(CHCl₃)δ(ppm) : 1.99 (1H, s), 3.05-3.15 (4H, m), 3.21-3.32 (4H, m), 7.13 (1H, d, J=1.9 Hz), 7.20-7.35 (2H, m), 7.35-7.50 (1H, m), 7.60-7.80 (3H, m)

### Referential Example 4

Piperazine (610 mg, 7.08 mmol) and 4-chloroquinoline (820 mg, 5.01 mmol) were added to pseudocumene (20 mL), and the mixture was refluxed for eight hours. Subsequently, the solvent contained in the reaction mixture was evaporated under reduced pressure. Aqueous 1N sodium hydroxide solution was added to the residue. Thereafter, the resultant mixture was extracted with chloroform, followed by drying over magnesium sulfate anhydrate and concentration under reduced pressure. The resultant residue was subjected to silica gel column chromatography, and the AcOEt:MeOH:Et₃N = 85:15:1 eluate was concentrated under reduced pressure, to thereby yield 738 mg of the product of interest, 1-(4-quinolyl)piperazine, as a pale yellow solid (yield 69.0%). MS(EI) m/z : 213 (M⁺)
¹H-NMR(CHCl₃)δ(ppm) : 1.73-1.96 (1H, br), 3.05-3.36 (8H, m), 6.77-6.88 (1H, m), 7.40-7.54 (1H, m), 7.58-7.70 (1H, m), 7.96-8.10 (2H, m), 8.67-8.76 (1H, m)

### Referential Example 5

1-(3-Methoxyphenyl)piperazine (3.0 g, 15.6 mmol) was added to 48% hydrobromic acid (25 mL), and the mixture was heated for five hours at 140°C. After cooling, the pH of the mixture was adjusted to 9 with aqueous 3N sodium hydroxide solution. Subsequently, the resultant mixture was extracted with chloroform, followed by drying over magnesium sulfate anhydrate. After concentration under reduced pressure, ether (50 mL) was added to the residue, and a pale red solid that precipitated was collected through filtration, to thereby yield 2.2 g of the product of interest, 1-(3-hydroxyphenyl)piperazine (yield 79.1%).
MS(FAB) m/z : 179 (M+H⁺)
¹H-NMR(DMSO)δ(ppm) : 2.70-2.85 (4H, m), 2.90-3.00 (4H, m), 6.18 (1H, dd, J =2.0,7.8 Hz), 6.27 (1H, s), 6.33 (1H, d, J=7.8Hz), 6.96 (1H, t, J=7.8 Hz), 9.00 (1H, br)

### Example 1

Dimethyl (tetrahydro-3, 3-diphenyl-2-furylidene) ammonium bromide (350 mg, 1.01 mmol) and 1-phenylpiperazine (150 mg, 0.899 mmol) were dissolved in anhydrous dimethylformamide (20 mL). Sodium carbonate (200 mg, 1.89 mmol) was added thereto, and the mixture was stirred for four hours at 110°C. Subsequently, the solvent was evaporated from the reaction mixture under reduced pressure. The resultant residue was dissolved in ethyl acetate, followed by washing with water, drying over magnesium sulfate anhydrate, and concentration under reduced pressure. The resultant residue was purified by silica gel chromatography. The 5% MeOH/AcOEt eluate was concentrated under reduced pressure, to thereby yield 373 mg of the product of interest, 4-(4-phenylpiperazin-1-yl)-N,N-dimethyl-2,2-diphenylbutanamide (Compound No. 1), as a pale yellow oil (yield 97.1%).

The thus-obtained compounds of interest described above were transformed into amorphous powder of hydrochloric acid salts through the below-described procedure, for the purpose of enhancing water solubility toward pharmacological testing. Moreover, Compound Nos. 2 to 51 obtained as described below were similarly processed, to thereby yield amorphous powder of hydrochloric acid salts, and subjected to pharmacological testing.

### (Production of hydrochloride)

The above-described product of interest (Compound No. 1, 360 mg, 0.834 mmol) was dissolved in anhydrous ether (30 mL). Under ice cooling, 1N hydrochloric acid-ether solution (0.9 mL) was added thereto for crystallization. The white precipitates that settled were collected through filtration, followed by washing with ether and drying, to thereby yield 330 mg of a hydrochloride of the above-described product of interest (i.e., a hydrochloride of Compound No. 1) (yield 84.5%).

### Examples 2 to 41, 46, and 47

A procedure similar to that of Example 1 was performed, whereby Compound Nos. 2 to 41, 46, and 47, shown in a relevant Table below, were obtained.

### Example 42

4-[4-(2-Hydroxyphenyl)piperazin-1-yl]-N,N-dimethyl-2,2-diphenylbutanamide (Compound No. 36) (1.1 g, 2.5 mmol) was dissolved in anhydrous dimethylformamide (20 mL). 2-Bromoethyl acetate (0.5 g, 3.0 mmol) and potassium carbonate (0.4 g, 3.0 mmol) were added thereto, and the mixture was stirred for 12 hours at room temperature. The reaction mixture was added to water, followed by extraction with ethyl acetate. The extract was washed with water and dried over magnesium sulfate anhydrate. The solvent was evaporated under reduced pressure, and the residue was purified by silica gel chromatography, to thereby yield 0.7 g of 2-[2-[4-[4-(dimethylamino)-3,3-diphenyl-4-oxobutyl]piperazin-1-yl]phenoxy]ethyl acetate (Compound No. 42) (yield 51.0%).

### Examples 44, 48, and 50

A procedure similar to that of Example 42 was performed, whereby Compound Nos. 44, 48, and 50, shown in a relevant Table below, were obtained.

### Example 43

2-[2-[4-[4-(Dimethylamino)-3,3-diphenyl-4-oxobutyl]piperazin-1-yl]phenoxy]ethyl acetate (Compound No. 42) (560 mg, 1.0 mmol) was dissolved in methanol (5 mL). Aqueous 1N sodium hydroxide solution (5 mL) was added, and the mixture was stirred for 1 hour at 50°C. Water was added to the reaction mixture, followed by extraction with chloroform, washing with water, and drying over sodium sulfate anhydrate. The solvent was evaporated under reduced pressure, and the residue was purified by silica gel chromatography, to thereby yield 440 mg of 4-[4-[2-(2-hydroxyethoxy)phenyl]piperazin-1-yl]-N,N-dimethyl-2,2-diphenylbutanamide (Compound No. 43) (yield 85.9%).

### Examples 45, 49, and 51

A procedure similar to that of Example 43 was performed, whereby Compound Nos. 45, 49, and 51, shown in a relevant Table below, were obtained.

### Example 52

4-[4-(2-Hydroxyphenyl)piperazin-1-yl]-N,N-dimethyl-2,2-diphenylbutanamide (Compound No. 36) (1.24 g, 2.80 mmol), ethyl α-bromoisobutyrate (6.00 g, 30.8 mmol), and potassium carbonate anhydrate (2.00 g, 14.5 mmol) were added to anhydrous dimethylformamide (20 mL), and the mixture was stirred for 16 hours at an external temperature of 50°C. After completion of reaction, dimethylformamide was evaporated under reduced pressure, and the residue was dissolved in ethyl acetate. The resultant mixture was washed with water and dried over sodium sulfate anhydrate. Subsequently, ethyl acetate was evaporated under reduced pressure, and the residual oily substance was subjected to column chromatography by use of silica gel (60 g). The 2% MeOH/CHCl₃ eluate was concentrated under reduced pressure, to thereby yield 820 mg of the product of interest, ethyl 2-[2-[4-[4-(dimethylamino)-3,3-diphenyl-4-oxobutyl]piperazin-1-yl]phenoxy]-2-methylpropionate (Compound No. 52), as a pale yellow oil (yield 52.6%).

### Example 53

Ethyl 2-[2-[4-[4-(dimethylamino)-3/3-diphenyl-4-oxobutyl]piperazin-1-yl]phenoxy]-2-methylpropionate (Compound No. 52)(660 mg, 1.18 mmol) was dissolved in a solvent mixture of aqueous 1N sodium hydroxide solution (10 mL), methanol (10 mL), and 1,4-dioxane (10 mL). The mixture was stirred for 3 hours at room temperature. After completion of reaction, the reaction mixture was added to water. The resultant mixture was neutralized to pH 7 with diluted hydrochloric acid, followed by extraction with chloroform, drying over sodium sulfate anhydrate. Chloroform was evaporated under reduced pressure, and the residual oily substance was crystallized with ether, to thereby yield 356 mg of the product of interest, ethyl 2-[2-[4-[4-(dimethylamino)-3,3-diphenyl-4-oxobutyl]piperazin-1-yl]phenoxy]-2-methylpropionate (Compound No. 53), as colorless crystals (yield 57.1%).

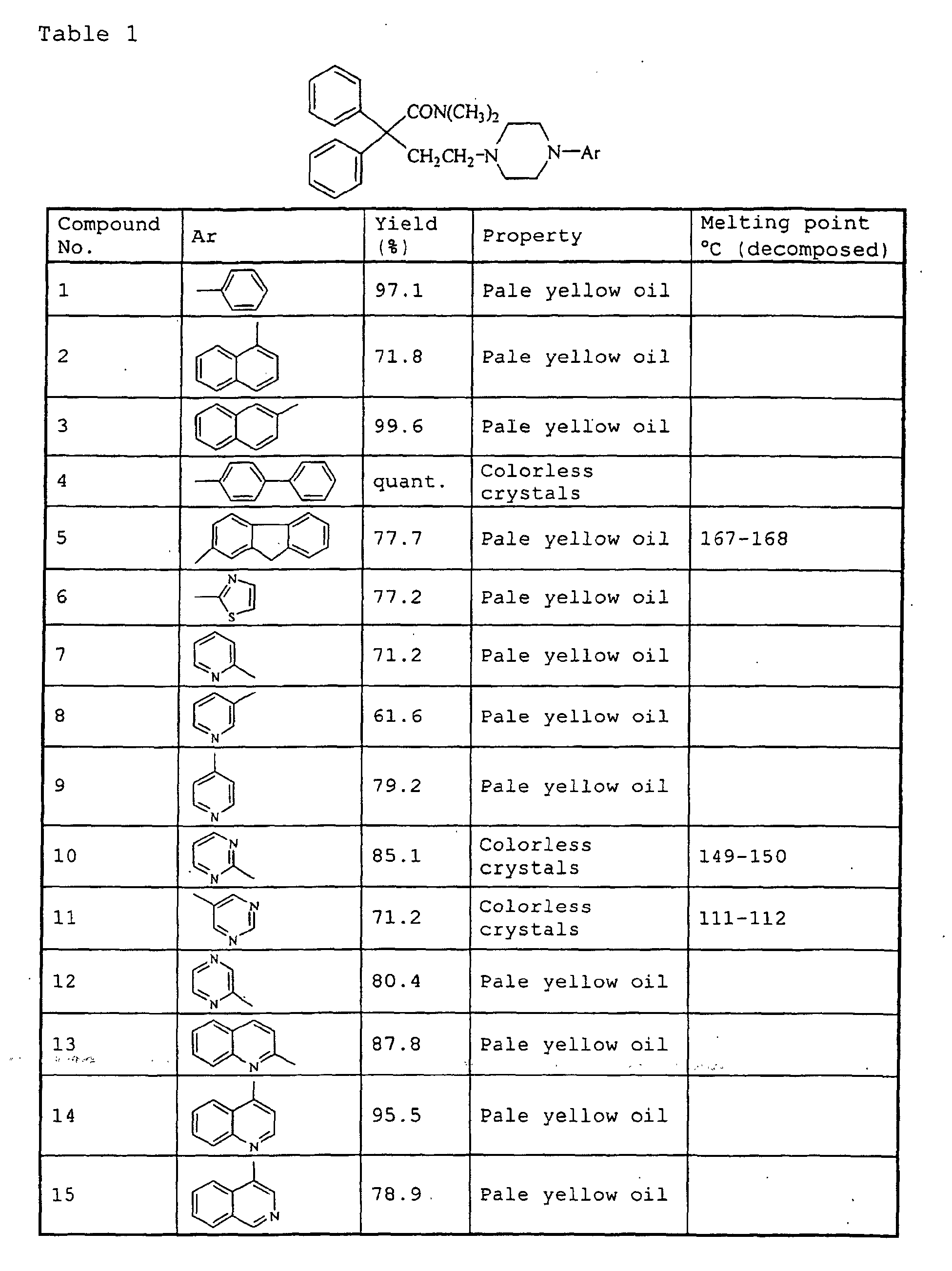

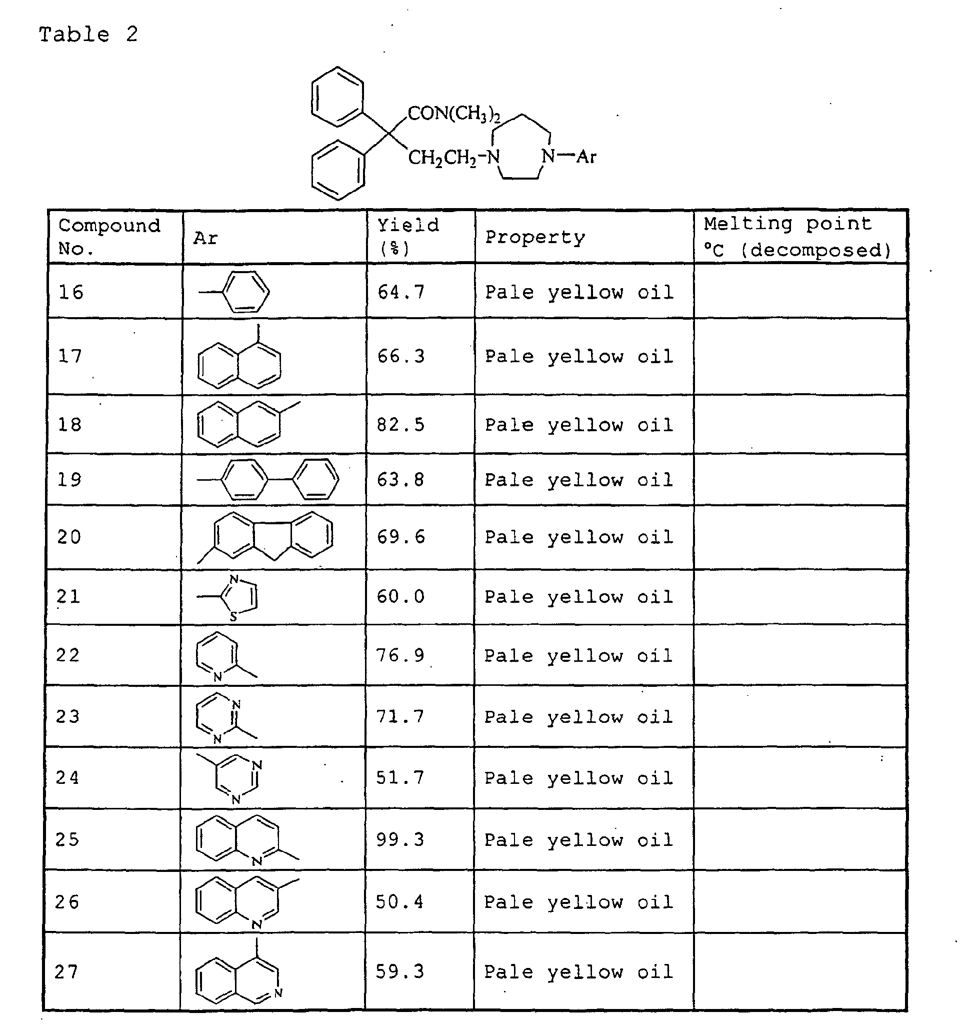

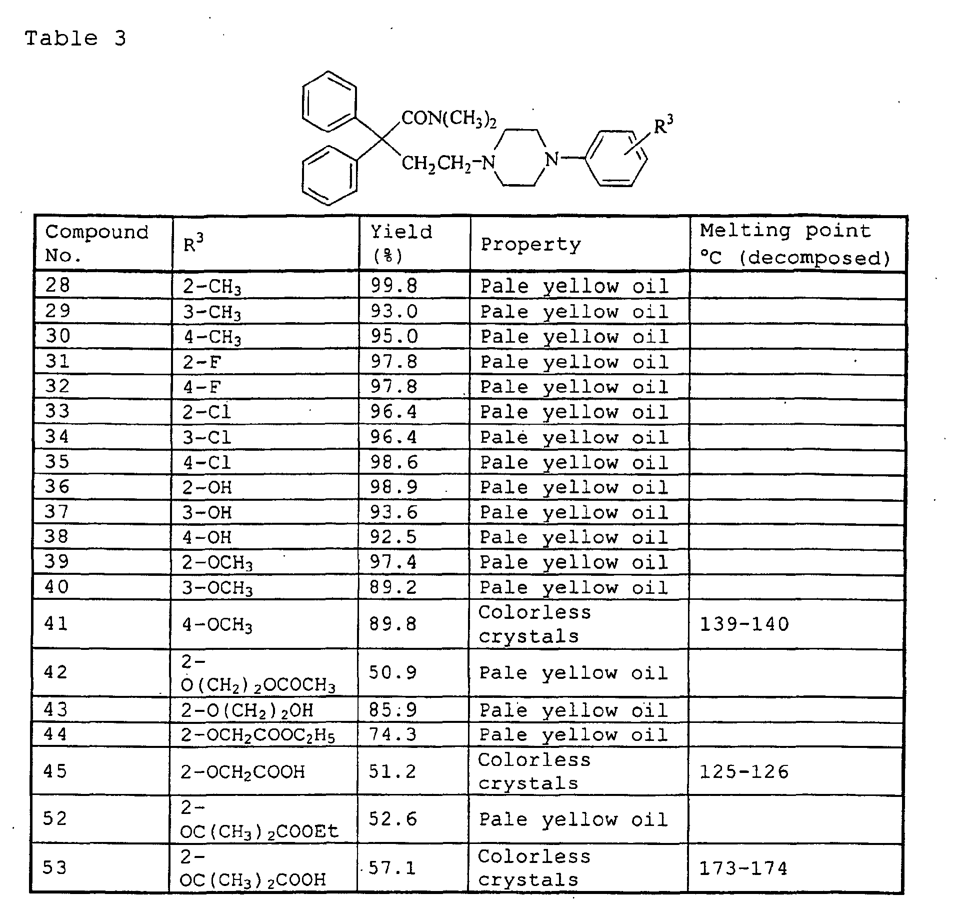

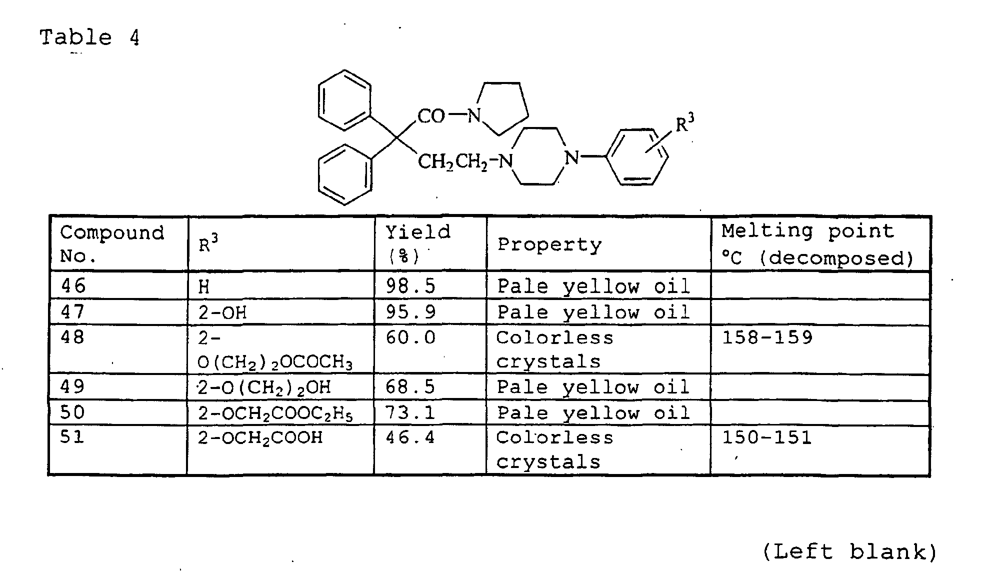

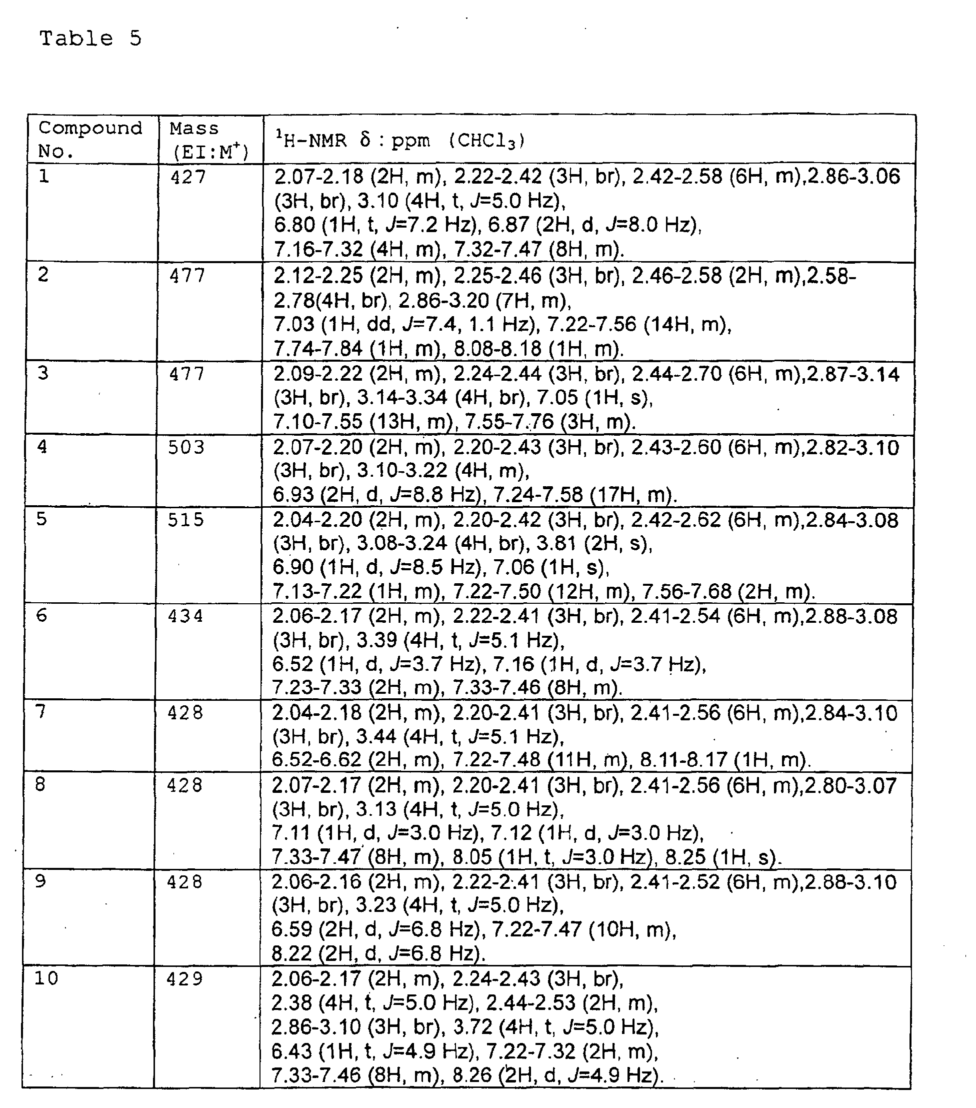

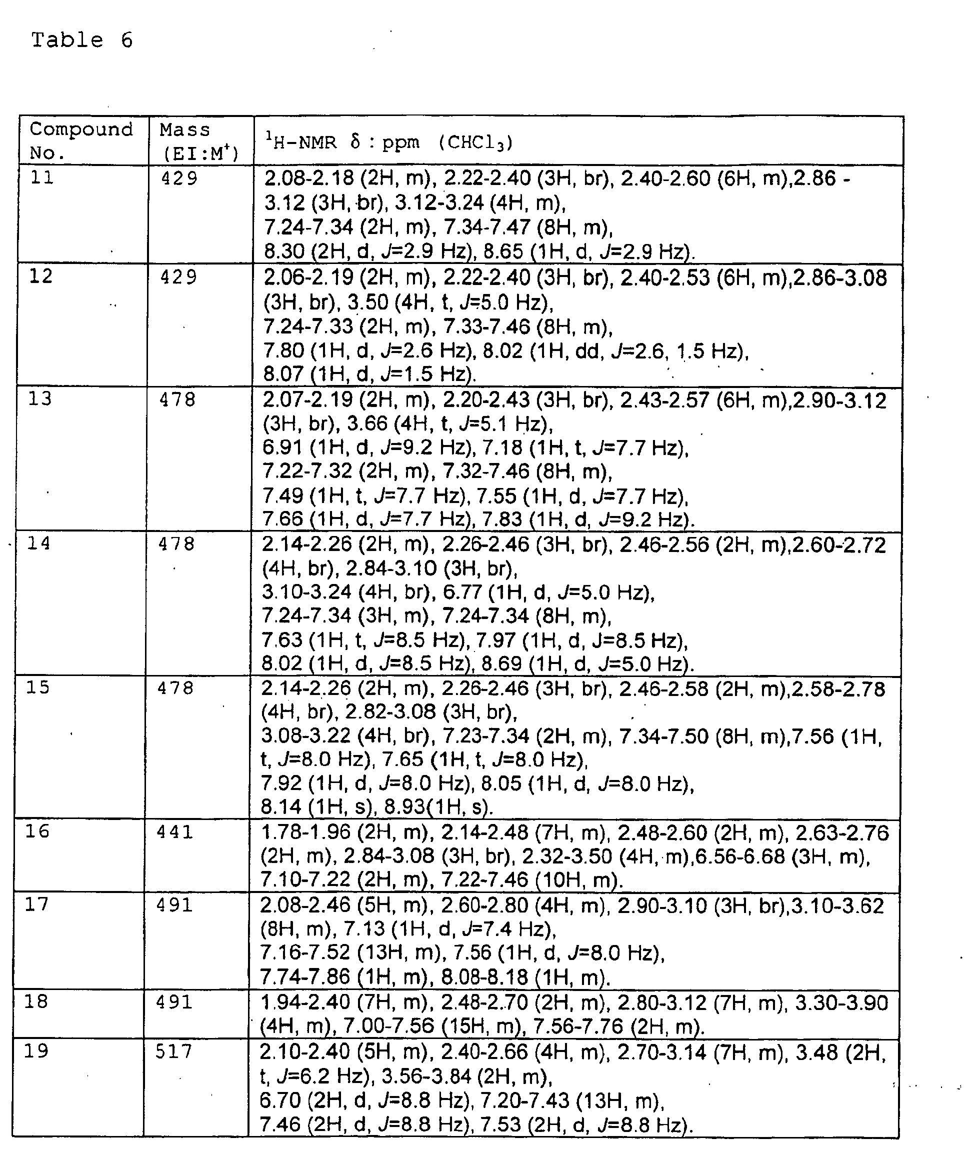

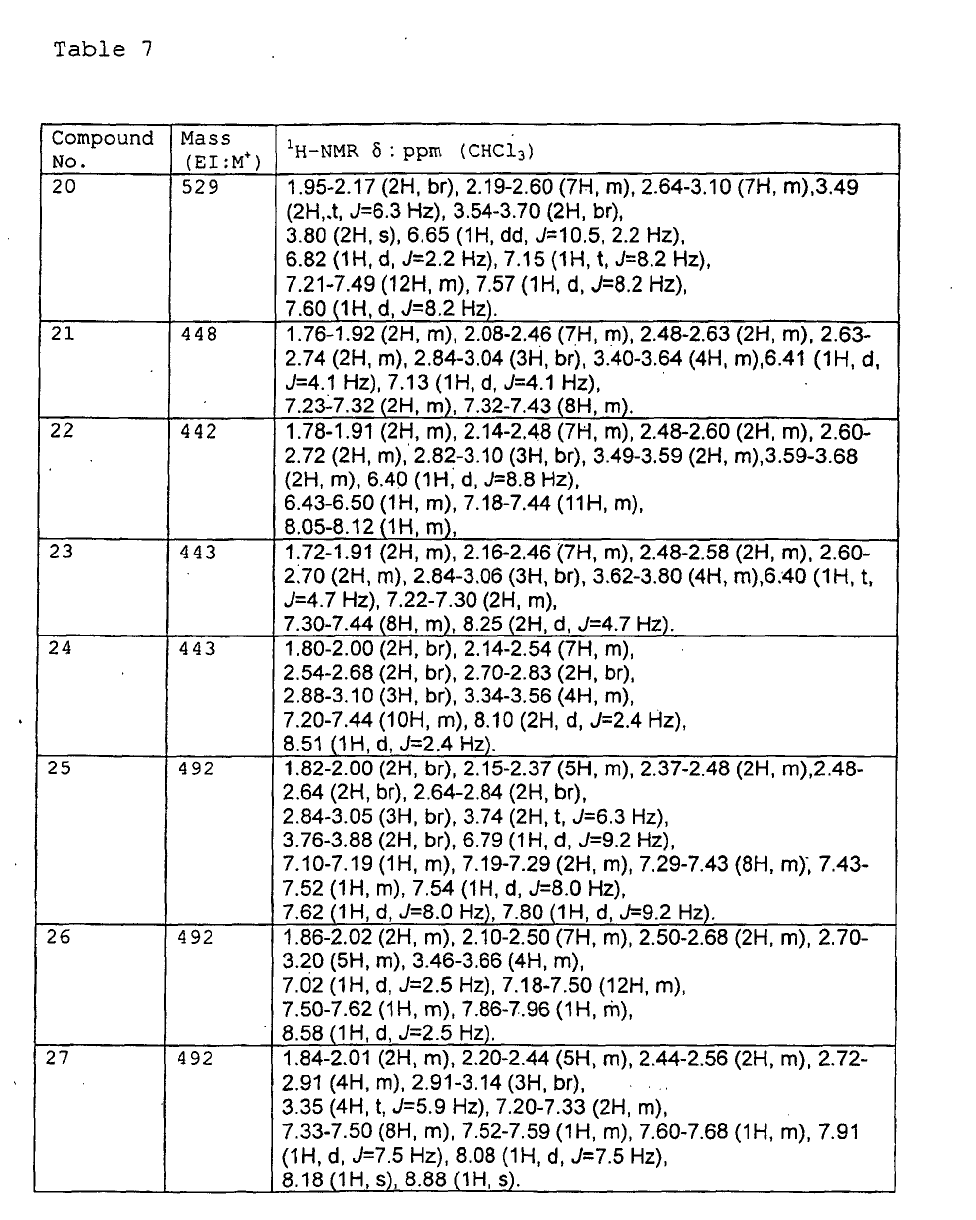

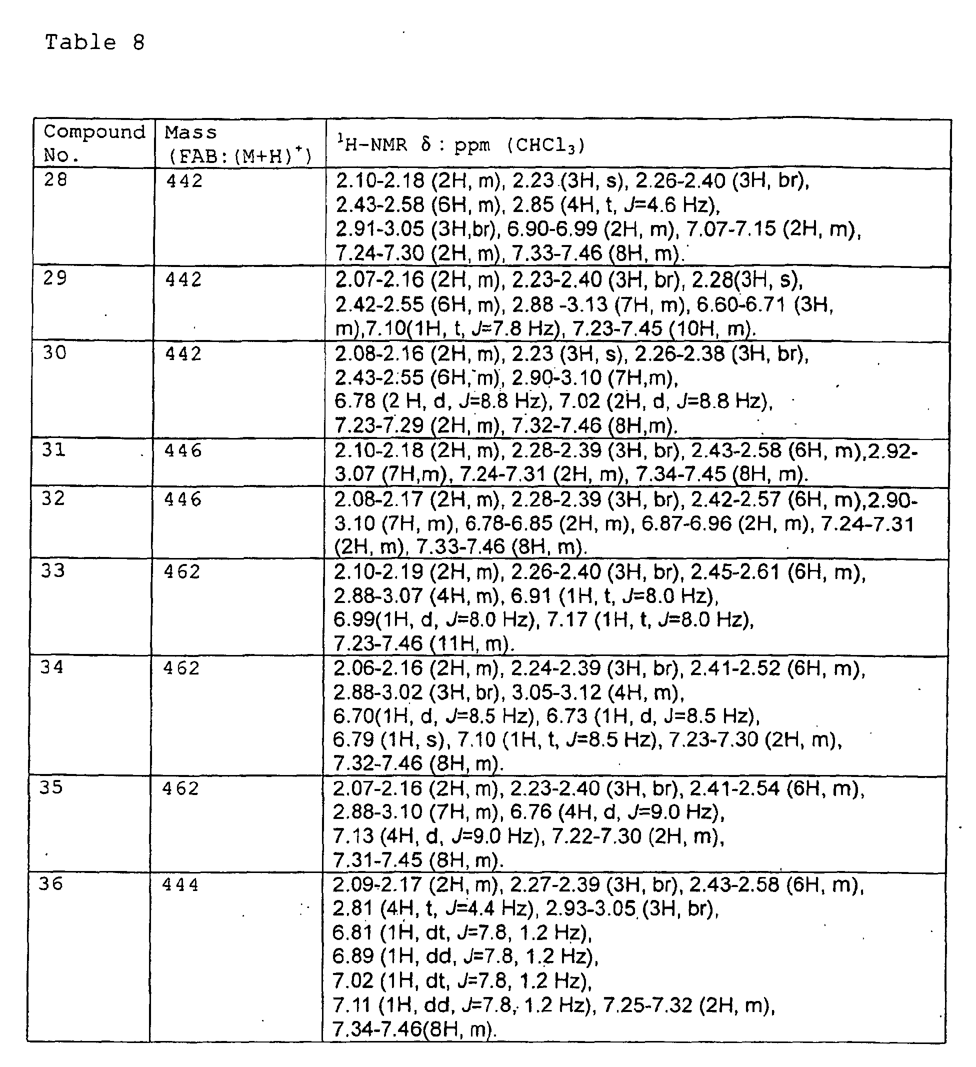

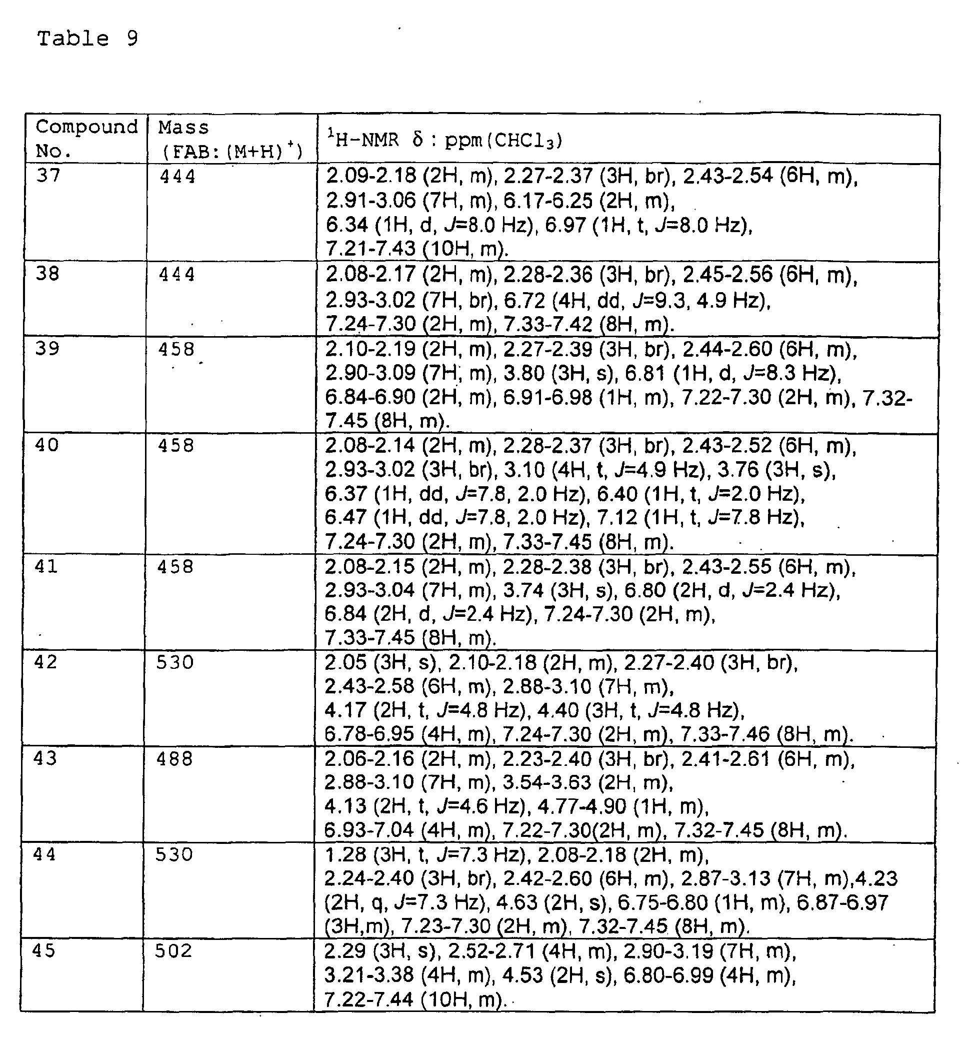

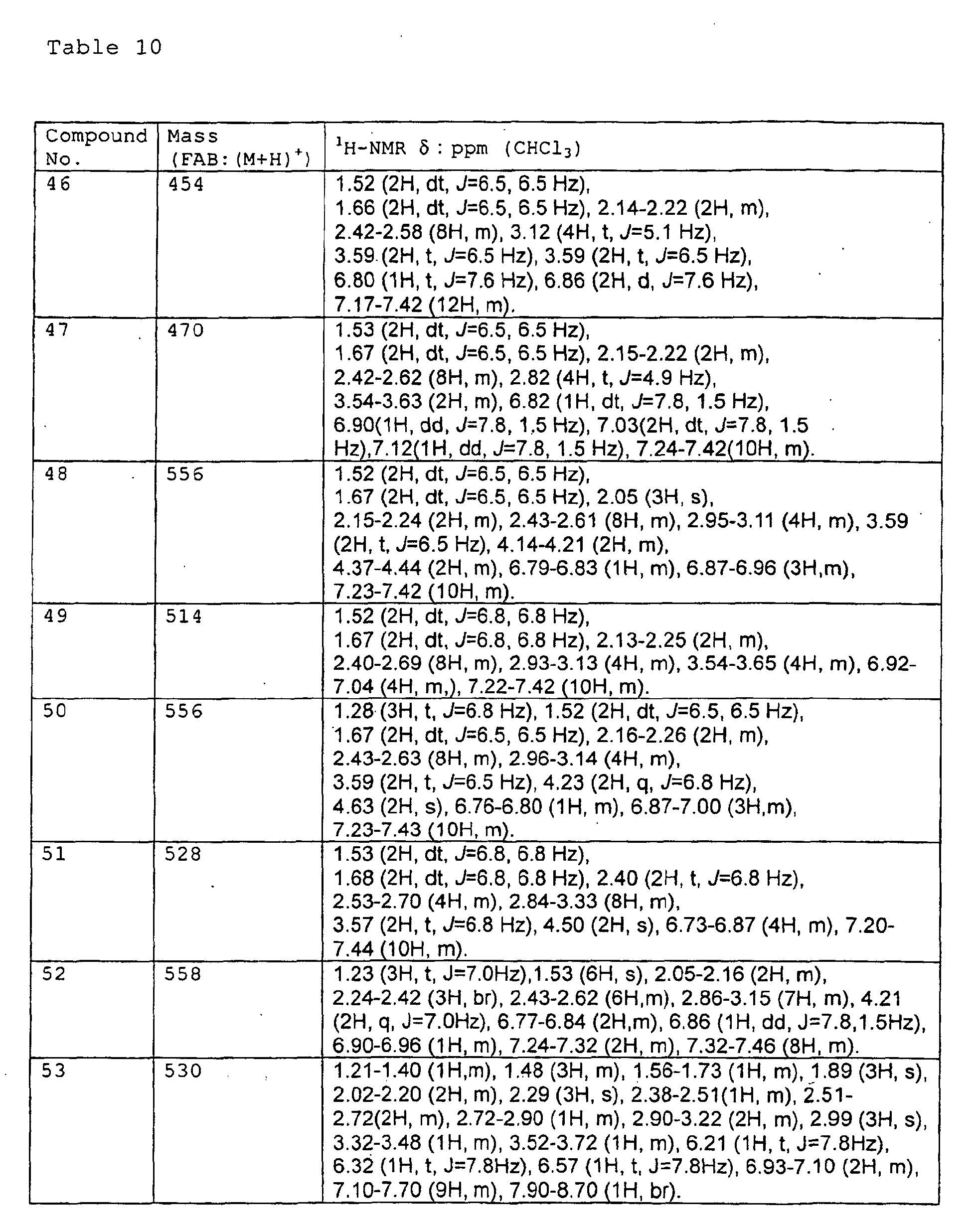

### Referential Example 6

4-Bromo-2,2-diphenylbutyric acid (23 g, 72 mmol) was suspended in chloroform (150 mL). Thionyl chloride (20 mL, 270 mmol) was added dropwise at room temperature. Subsequently, dimethylformamide (DMF; 0.2 mL) was added, and the mixture was heated and refluxed for 4 hours. Thereafter, the solvent was evaporated under reduced pressure, to thereby yield 23 g of 4-bromo-2,2-diphenylbutyric chloride (yield 94.7%).

Aqueous 50% dimethylamine solution (8 g, 90 mmol) and sodium carbonate (18 g, 170 mmol) were suspended in water (100 mL), and the suspension was cooled to a temperature between 0 and 5°C. To the cooled suspension was added dropwise the above-prepared 4-bromo-2,2-diphenylbutyric chloride (23 g, 68 mmol) dissolved in toluene (100 mL). The mixture was stirred for 2 hours, and thereafter, the aqueous layer was washed with toluene, followed by extraction with chloroform, washing with water, and drying. The solvent was evaporated under reduced pressure, and the residue was crystallized from methyl isobutyl ketone, to thereby yield 11 g of dimethyl(tetrahydro-3,3-diphenyl-2-furylidene)ammonium bromide (yield 46.8%).
¹H-NMR(CDCl₃)δ(ppm) :
2.96 (3H, s), 3.47 (2H, t, J=6.4Hz), 3.83 (3H, s), 4.85 (2H, t, J=6.4Hz), 7.40-7.60 (10H, m)

The procedure of Referential Example 6 was repeated except that pyrrolidine was used instead of aqueous 50% dimethylamine solution, whereby (tetrahydro-3,3-diphenyl-2-furylidene)pyrrolidinium bromide was obtained (yield 53.6%). ¹H-NMR(CDCl₃)δ(ppm) :
1.96 (2H, quintet, J=6.4Hz), 2.13 (2H, quintet, J=7.4Hz), 2.89 (2H, t, J=6.8Hz), 3.50 (2H, t, J=6.4Hz), 4.37 (2H, t, J=7.3H z), 4.87 (2H, t, J=6.4Hz), 7.40-7.55 (10H, m)

### Referential Example 7

Magnesium powder (240 mg, 10 mmol) was added to anhydrous tetrahydrofuran (2 mL). Separately, 2-methoxybromobenzene (1.9 g, 10.7 mmol) was dissolved in anhydrous tetrahydrofuran (10 mL), and an aliquot (one fifth the amount) of the resultant mixture was added. Thereafter, 1, 2-dibromoethane (0.1 mL) and a small amount of iodine were added thereto, and the mixture was heated to 40°C. After reaction started, the remaining portion of the solution of 2-methoxybromobenzene in anhydrous tetrahydrofuran was added dropwise, and the mixture was heated and refluxed for 30 minutes.

1-Benzyl-4-piperidone (1.89 g, 10 mmol) was dissolved in anhydrous tetrahydrofuran (30 mL) at room temperature, and the mixture was added dropwise to the obtained solution of Grignard reagent in anhydrous tetrahydrofuran. Subsequently, the resultant mixture was heated and refluxed for 30 minutes, and aqueous saturated ammonium chloride solution (10 mL) was added dropwise. Thereafter, the solvent was concentrated under reduced pressure. Water was added to the residue, followed by extraction with ether, washing with water, and drying. The solvent was evaporated under reduced pressure, and the residue was purified by silica gel chromatography, to thereby yield 1.84 g of 1-benzyl-4-(2-methoxyphenyl)-4-piperidinol (yield 61.8%).
¹H-NMR(CDCl₃)δ(ppm) :
1.50-1.90 (2H, m), 2.52 (2H, dt, J=12.8Hz, 4.8Hz), 2.80-3.25 (3H, m), 3.25-3.50 (2H, m), 3.87 (3H, s), 6.91 (1H, dd, J=,1.1 Hz), 7.03 (1H, s), 7.10-7.40 (1H, m), 7.40-7.60 (1H, m)

### Referential Example 8

1-Benzyl-4-(2-methoxyphenyl)-4-piperidinol (2.08 g, 7.0 mmol) was dissolved in dioxane (10 mL). Subsequently, 6N hydrochloric acid (20 mL) was added thereto, and the mixture was heated and refluxed for 3 hours. After completion of reaction, the pH of the mixture was adjusted to 9 with aqueous 1N sodium hydroxide solution, followed by extraction with ethyl acetate, washing with water, and drying. The solvent was evaporated under reduced pressure, and the residue was purified by silica gel chromatography, to thereby yield 1.50 g of 1-benzyl-4-(2-methoxyphenyl)-1,2,3,6-tetrahydropyridine (yield 77.0%).
¹H-NMR(CDCl₃)δ(ppm) :
2.50-2.58 (2H, m), 2.67 (2H, t, J=5.9Hz), 3.15-3.19 (2H, m), 3. 65 (2H, s), 3.80 (3H, s), 5.75-5.78 (1H, m), 6.85 (1H, d, J=8.3 Hz), 6.89 (1H, dt, J=8.3Hz, 1.0Hz), 7.15-7.42 (7H, m)

### Referential Example 9

1-Benzyl-4-(2-methoxyphenyl)-1,2,3,6-tetrahydropyridine (1.5 g, 5.4 mmol) was dissolved in ethanol (30 mL). Subsequently, 10% palladium-carbon (0.6 g) was added thereto, and the mixture was subjected to catalytic reduction at room temperature at 1 atm for 6 hours. After completion of reaction, the catalyst was filtered off, and the filtrate was concentrated under reduced pressure, to thereby yield 0.88 g of 4-(2-methoxyphenyl)piperidine (yield 85.7%).
¹H-NMR(CDCl₃)δ(ppm) :
1.95-2.05 (2H, m), 2.10-2.23 (2H, m), 3.04 (2H, t, J=10.2Hz), 3.15-3.25 (1H, m), 3.60-3.68 (2H, m), 3.84 (3H, s), 6.87 (1H, d, J=8.3Hz), 6.94 (1H, t, J=6.4Hz), 7.17-7.25 (2H, m)

### Referential Example 10

4-(2-Methoxyphenyl)piperidine (530 mg, 2.3 mmol) was added to 48% hydrobromic acid (10 mL), and the mixture was heated and stirred for 17 hours at 140°C. After cooling, aqueous 3N sodium hydroxide solution was added to the reaction mixture, whereby the pH of the mixture was adjusted to 9, followed by extraction with chloroform, drying over magnesium sulfate anhydrate, and concentration under reduced pressure, to thereby yield 4-(2-hydroxyphenyl)piperidine in powder form as a residue (280 mg, 68.0%).
¹H-NMR(CDCl₃)δ(ppm) :
1.70-1.93 (4H, m), 2.70-2.90 (2H, m), 2.95-3.10 (1H, m), 3.10-3.32 (2H, m), 4.00-5.20 (2H, br), 6.70 (1H, dd, J=7.7Hz, 1.4H z), 6.84 (1H, dt, J=7.7Hz, 1.4Hz), 7.04 (1H, dt, J=7.7Hz, 1.4H z), 7.14 (1H, dd, J=7.7Hz, 1.4Hz)

### Referential Example 11

Dimethyl (tetrahydro-3,3-diphenyl-2-furylidene)ammonium bromide (3.5 g, 10 mmol) and 4-(2-hydroxyphenyl)-4-piperidinol (1.93 g, 10 mmol) were dissolved in anhydrous dimethylformamide (20 mL) in the presence of sodium carbonate (3.2 g, 30 mmol), and the mixture was stirred for 3 hours at 100°C. The reaction mixture was added to water, followed by extraction with ethyl acetate, washing with water, and drying. The solvent was evaporated under reduced pressure, and the residue was purified by silica gel chromatography, to thereby yield 3.6 g of 4-[4-(2-hydroxyphenyl)-4-hydroxypiperidin-1-yl]-2,2-diphenyl-N,N-dimethylbutanamide (yield 78.6%).

### Referential Example 12

(Tetrahydro-3,3-diphenyl-2-furylidene)pyrrolidinium bromide (10.75 g, 29 mmol) and 4-(2-hydroxyphenyl)-4-piperidinol (6.20 g, 32 mmol) were dissolved in anhydrous dimethylformamide (200 mL) in the presence of sodium carbonate (8.5 g, 80 mmol), and the mixture was stirred for 3 hours at 100°C. The reaction mixture was added to water, followed by extraction with ethyl acetate, washing with water, and drying. The solvent was evaporated under reduced pressure, and the residue was purified by silica gel chromatography, to thereby yield 13.9 g of 4-[4-(2-hydroxyphenyl)-4-hydroxypiperidin-1-yl]-2,2-diphenyl-1-(1-pyrrolidinyl)-1-butanone (yield 99.0%).

### Example 54

Dimethyl (tetrahydro-3,3-diphenyl-2-furylidene)ammonium bromide (500 mg, 1.4 mmol) and 4-(2-methoxyphenyl)piperidine (300 mg, 1.6 mmol) were dissolved in anhydrous dimethylformamide (20 mL). Subsequently, sodium carbonate (500 mg, 5 mmol) was added, and the mixture was stirred for 3 hours at 100°C. The reaction mixture was added to water, followed by extraction with ethyl acetate, washing with water, and drying. The solvent was evaporated under reduced pressure, and the residue was purified by silica gel chromatography, to thereby yield 440 mg of 4-[4-(2-methoxyphenyl)piperidin-1-yl]-N,N-dimethyl-2,2-diphenylbutanamide (Compound No. 54) (yield 66.9%).

### Examples 55 to 64

A procedure similar to that of Example 1 was performed, whereby Compound Nos. 55 to 64 were obtained.

### Example 65

4-[4-(2-Hydroxyphenyl)piperidin-1-yl]-N,N-dimethyl-2,2-diphenylbutanamide (Compound No. 57) (580 mg, 1.3 mmol) was dissolved in anhydrous dimethylformamide (12 mL), and 100 mg of potassium hydroxide (powder) was added. Ethyl chloroacetate (180 mg, 1.5 mmol) was added dropwise at 0°C, and the resultant mixture was stirred for 12 hours at room temperature. The reaction mixture was added to water, followed by extraction with ethyl acetate, washing with water, and drying. The solvent was evaporated under reduced pressure, and the residue was purified by silica gel chromatography, to thereby yield 395 mg of ethyl [2-[1-[4-(dimethylamino)-3,3-diphenyl-4-oxobutyl]piperidin-4-yl]phenoxy]acetate (Compound No. 65) (yield 57.0%).

### Example 66

4-[4-(2-Hydroxyphenyl)piperidin-1-yl]-N,N-dimethyl-2,2-diphenylbutanamide (Compound No. 57) (1,500 mg, 3.4 mmol) was dissolved in anhydrous dimethylformamide (20 mL). Subsequently, 2-bromoethyl acetate (600 mg, 3.6 mmol), potassium carbonate (600 mg, 4.5 mmol), and a small amount of potassium iodide were added, and the resultant mixture was stirred for 12 hours at room temperature. The reaction mixture was added to water, followed by extraction with ethyl acetate, washing with water, and drying. The solvent was evaporated under reduced pressure, and the residue was purified by silica gel chromatography, to thereby yield 390 mg of 2-[2-[1-[4-(dimethylamino)-3,3-diphenyl-4-oxobutyl]piperidin-4-yl]phenoxy]ethyl acetate (Compound No. 66) (yield 21.6%).

### Examples 67 and 68

A procedure similar to that of Example 66 was performed, whereby Compound Nos. 67 and 68 were obtained.

### Example 69

2-[2-[1-[4-(Dimethylamino)-3,3-diphenyl-4-oxobutyl]piperidin-4-yl]phenoxy]ethyl acetate (Compound No. 66) (360 mg, 0.68 mmol) was dissolved in methanol (5 mL). Subsequently, aqueous 1N sodium hydroxide solution (1 mL) was added, and the mixture was stirred for 1 hour at 50°C. Water was added to the reaction mixture, followed by extraction with chloroform, washing with water, and drying. The solvent was evaporated under reduced pressure, and the residue was purified by silica gel chromatography, to thereby yield 203 mg of 4-[4-[2-(2-hydroxyethoxy)phenyl]piperidin-1-yl]-N,N-dimethyl-2,2-diphenylbutanamide (Compound No. 69) (yield 61.3%).

### Examples 70 and 71

A procedure similar to that of Example 69 was performed, whereby Compound Nos. 70 and 71 were obtained.

### Example 72

Ethyl [2-[1-[4-(dimethylamino)-3,3-diphenyl-4-oxobutyl]piperidin-4-yl]phenoxy]acetate (Compound No. 65) (360 mg, 0.7 mmol) was dissolved in methanol (5 mL). Subsequently, aqueous 1N sodium hydroxide solution (5 mL) was added, and the mixture was stirred for 1 hour at 50°C. After completion of reaction, the reaction mixture was added to water, and the pH of the mixture was adjusted to 4 to 5 with 1N HCl, followed by extraction with chloroform, washing with water, and drying. The solvent was evaporated under reduced pressure, and the residue was purified by silica gel chromatography, to thereby yield 320 mg of [2-[1-[4-(dimethylamino)3,3-diphenyl-4-oxobutyl]piperidin-4-yl]phenoxy]acetic acid (Compound No. 72) (yield 94.0%).

### Example 73

4-[4-(2-Hydroxyphenyl)-4-hydroxypiperidin-1-yl]-2,2-diphenyl-N,N-dimethylbutanamide (1.40 g, 3.1 mmol) was dissolved in a solvent mixture of dioxane (7 mL) and 6N hydrochloric acid (14 mL), and the mixture was heated and refluxed for 3 hours. After completion of reaction, and the pH of the mixture was adjusted to 11 with aqueous 1N sodium hydroxide solution, followed by extraction with ethyl acetate, washing with water, and drying. The solvent was evaporated under reduced pressure, and the residue was purified by silica gel chromatography, to thereby yield 1.24 g of 4-[4-(2-hydroxyphenyl)-1,2,3,6-tetrahydropyridin-1-yl]-N,N-dimethyl-2,2-diphenylbutanamide (Compound No. 73) (yield 92.3%).

### Example 74

The procedure of Example 65 was repeated by use of 4-(4-(2-hydroxyphenyl)-1,2,3,6-tetrahydropyridin-1-yl)-N,N-dimethyl-2,2-diphenylbutanamide (Compound No. 73), whereby ethyl [2-[1-[4-(dimethylamino)-3,3-diphenyl-4-oxobutyl]-1,2,3,6-tetrahydropyridin-4-yl]phenoxy]acetate (Compound No. 74) was obtained.

### Example 75

The procedure of Example 66 was repeated by use of Compound No. 73, and without isolating the resultant product, a procedure similar to that of Example 69 was performed, whereby 4-[4-[2-(2-hydroxyethoxy)phenyl]-1,2,3,6-tetrahydropyridin-1-yl]-N,N-dimethyl-2,2-diphenylbutanamide (Compound No. 75) was obtained.

### Example 76

The procedure of Example 72 was repeated by use of ethyl [2-[1-[4-(dimethylamino)-3,3-diphenyl-4-oxobutyl]-1,2,3,6-tetrahydropyridin-4-yl]phenoxy]acetate (Compound No. 74), whereby [2-[1-[4-(dimethylamino)-3,3-diphenyl-4-oxobutyl]-1,2,3,6-tetrahydropyridin-4-yl]phenoxy]acetic acid (Compound No. 76) was obtained.

### Example 77

4-[4-(2-Hydroxyphenyl)-4-hydroxypiperidin-1-yl]-2,2-diphenyl-1-(1-pyrrolidyl)-1-butanone (4.20 g, 8.7 mmol) was dissolved in a solvent mixture of dioxane (20 mL) and 6N hydrochloric acid (40 mL), and the mixture was heated and refluxed for 3 hours. After completion of reaction, the pH of the mixture was adjusted to 11 with aqueous 1N sodium hydroxide solution, followed by extraction with ethyl acetate, washing with water, and drying. The solvent was evaporated under reduced pressure, and the residue was purified by silica gel chromatography, to thereby yield 3.45 g of 4-[4-(2-hydroxyphenyl)-1,2,3,6-tetrahydropyridin-1-yl]-2,2-diphenyl-1-(1-pyrrolidinyl)-1-butanone (Compound No. 77) (yield 85.3%).

### Example 78

4-[4-(2-Hydroxyphenyl)-1,2,3,6-tetrahydropyridin-1-yl]-2,2-diphenyl-1-(1-pyrrolidinyl)-1-butanone (Compound No. 77) (2.50 g, 5.36 mmol) was dissolved in ethanol (80 mL), and 10% palladium-carbon (0.80 g) was added thereto. The mixture was subjected to catalytic reduction in a hydrogen atmosphere at room temperature at 1 atm for 12 hours. After completion of reaction, the catalyst was filtered off, and the filtrate was concentrated under reduced pressure, to thereby yield 1.45 g of 4-[4-(2-hydroxyphenyl)piperidin-1-yl]-2,2-diphenyl-1-(1-pyrrolidinyl)-1-butanone (Compound No. 78) (yield 60.3%)

### Example 79

The procedure of Example 65 was repeated by use of 4-[4-(2-hydroxyphenyl)piperidin-1-yl]-2,2-diphenyl-1-(1-pyrrolidyl)-1-butanone (Compound No. 78), whereby [2-[1-[4-(1-pyrrolidinyl)-3,3-diphenyl-4-oxobutyl]piperidin-4-yl]phenoxy]ethyl acetate (Compound No. 79) was obtained.

### Example 80

The procedure of Example 66 was repeated by use of 4-[4-(2-hydroxyphenyl)piperidin-1-yl]-2,2-diphenyl-1-(1-pyrrolidyl)-1-butanone (Compound No. 78), whereby 2-[2-[1-[3,3-diphenyl-4-oxo-4-(1-pyrrolidinyl)butyl]piperidin-4-yl]phenoxy]ethyl acetate (Compound No. 80) was obtained.

### Example 81

The procedure of Example 69 was repeated by use of 2-[2-[1-[3,3-diphenyl-4-oxo-4-(1-pyrrolidinyl)butyl]piperidin-4-yl]phenoxy]ethyl acetate (Compound No. 80), whereby 4-[4-[2-(2-hydroxyethoxy)phenyl]piperidin-1-yl]-2,2-diphenyl-1-(1-pyrrolidinyl)-1-butanone (Compound No. 81) was obtained.

### Example 82

The procedure of Example 72 was repeated by use of ethyl [2-[1-[4-(1-pyrrolidinyl)-3,3-diphenyl-4-oxobutyl]piperidin-4-yl]phenoxy]acetate (Compound No. 79), whereby [2-[1-[4-(1-pyrrolidinyl)-3,3-diphenyl-4-oxobutyl]piperidin-4-yl]phenoxy]acetic acid (Compound No. 82) was obtained.

### Example 83

4-[4-(2-Hydroxyphenyl)piperidin-l-yl]-N,N-dimethyl-2,2-diphenylbutanamide (Compound No. 57) (400 mg, 0.905 mmol), ethyl α-bromoisobutyrate (2.00 g, 10.3 mmol), and potassium carbonate anhydrate (700 mg, 5.06 mmol) were added to anhydrous dimethylformamide (10 mL), and the mixture was stirred for 15 hours at an external temperature of 50°C. After completion of reaction, dimethylformamide was evaporated under reduced pressure, and the residue was dissolved in ethyl acetate, followed by washing with water and drying over sodium sulfate anhydrate. Subsequently, ethyl acetate was evaporated under reduced pressure, and the residual oily substance was subjected to column chromatography by use of silica gel (60 g). The 3% MeOH/CHCl₃ eluate was concentrated under reduced pressure, to thereby yield 181 mg of the product of interest, ethyl 2-[2-[1-[4-(dimethylamino)-3,3-diphenyl-4-oxobutyl]piperidin-4-yl]phenoxy]-2-methylpropionate (Compound No. 83), as a pale yellow oil (yield 36.0%).

### Example 84

Ethyl 2-[2-[1-[4-(dimethylamino)-3,3-diphenyl-4-oxobutyl]piperidin-4-yl]phenoxy]-2-methylpropionate (Compound No. 83) (240 mg, 0.432 mmol) was dissolved in a solvent mixture of aqueous 1N sodium hydroxide solution (5 mL), methanol (5 mL), and 1,4-dioxane (5 mL), and the mixture was stirred for 5 hours at room temperature. After completion of reaction, the reaction mixture was added to water, and the resultant mixture was neutralized to pH 7 with diluted hydrochloric acid, followed by extraction with chloroform and drying over sodium sulfate anhydrate. Chloroform was evaporated under reduced pressure, and the precipitated crystals were pulverized in the presence of ether, to thereby yield 169 mg of the product of interest, 2-[2-[1-[4-(dimethylamino)-3,3-diphenyl-4-oxobutyl]piperidin-4-yl]phenoxy]-2-methylpropionic acid (Compound No. 84), as colorless crystals (yield 74.1%).

Tables 11 to 14 show the yield, property, and melting point of Compounds No. 54 to 84, and Tables 15 to 17 show NMR data of the compounds.

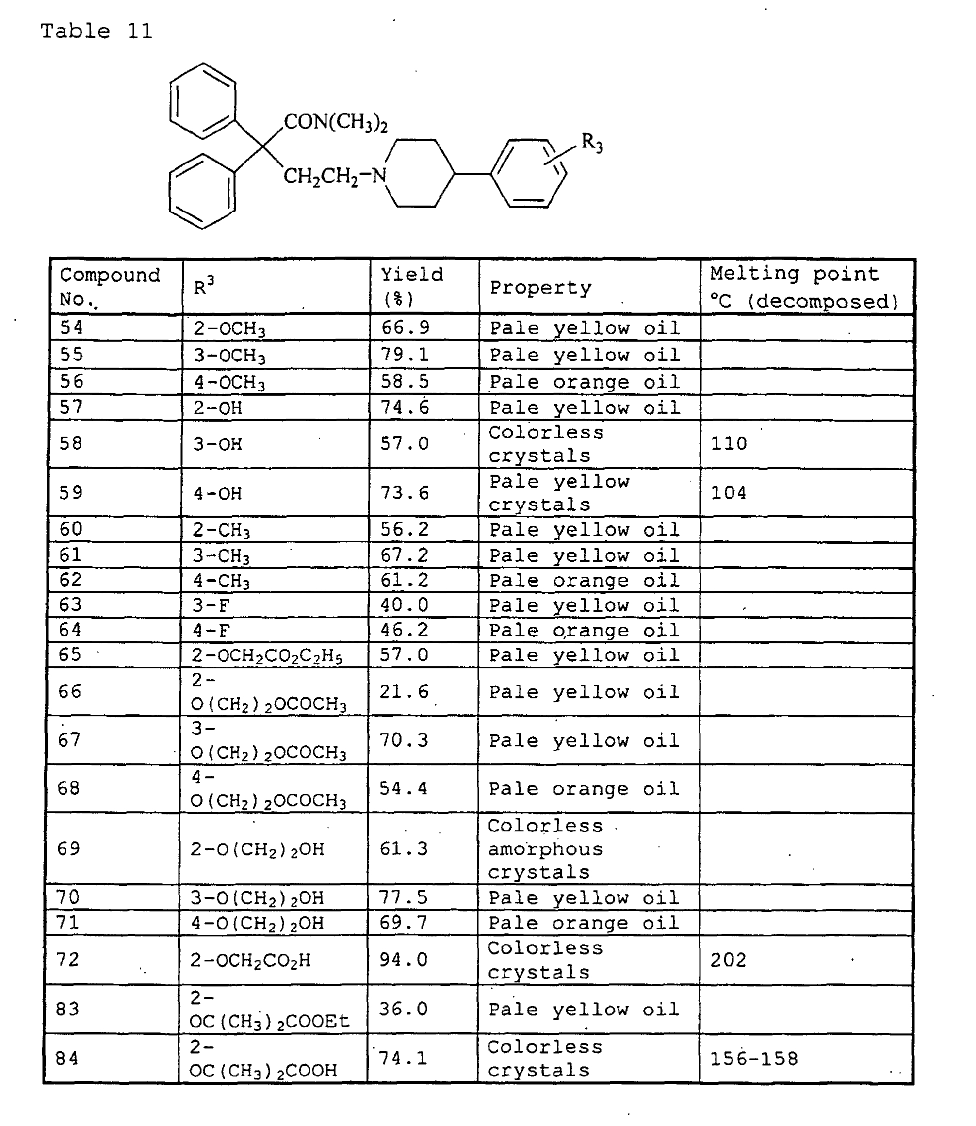

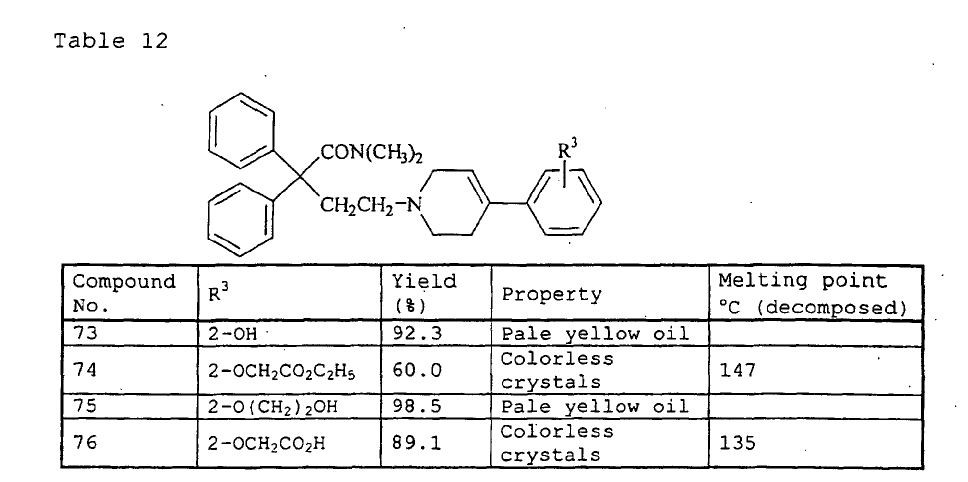

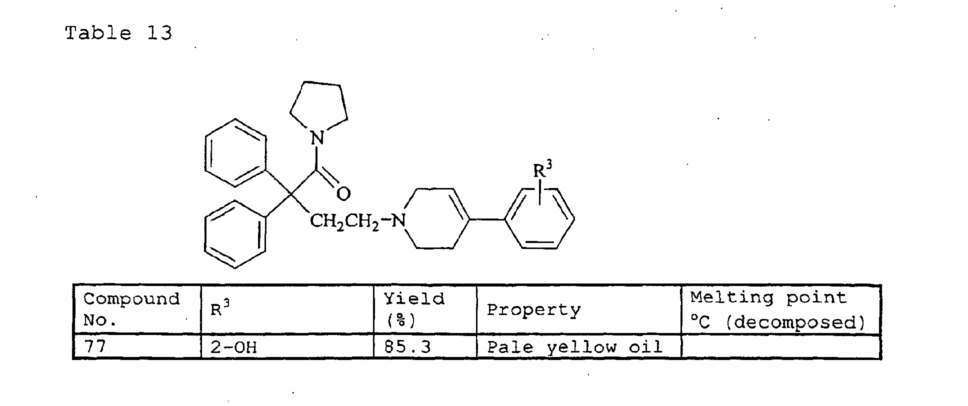

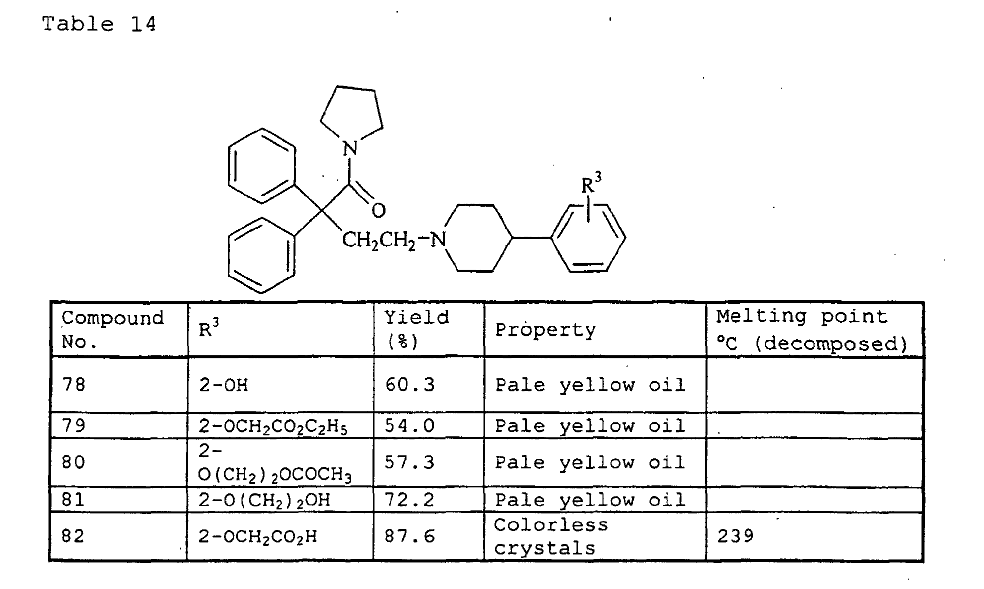

**Table 15**

| Compound No. | Mass (FAB: (M+H)⁺) | ¹H-NMR δ : ppm (CDCl₃) |
|---|---|---|
| 54 | 457 | 1.58-1.80(4H, m), 1.94-2.20(4H, m), 2.24-2.39(3H, br), 2.44-2.58(2H, m), 2.80-3.06(6H, m), 3.78(3H, s), 6.78-6.92(2H, m), 7.12-7.19(2H, m), 7.24-7.44(10H, m). |
| 55 | 457 | 1.63-1.80(4H, m), 1.90-2.05(3H, br), 2.08-2.17(2H, m), 24-2.42(4H, m), 2.47-2.55(2H, m), 2.88-3.08(3H, m), 3.76(3H, s), 6.68-6.74(2H, m), 6.77(1H, d, J=7.8Hz), 7.17(1H, t, J=7.8Hz), 7.25-7.45(10H, m). |
| 56 | 457 | 1.62-1.77(4H, m), 1.77-1.93(2H, m), 1.93-2.07(2H, m), 2.07-2.20(2H, m), 2.25-2.42(3H, m), 2.46-2.57(2H, m), 2.90-3.10(4H, m), 3.76(3H, s), 6.81(2H, d, J=8.8Hz), 7.10(2H, d, J=8.8Hz), 7.25-7.30(2H, m), 7.34-7.45(8H, m). |
| 57 | 443 | 1.77-1.88(2H, m), 2.06-2.22(2H, m), 2.31(3H, s), 2.52-2.79(6H, m), 2.90-3.04(1H, m), 3.01(3H, s), 3.24-3.37(2H, m), 6.72-6.78(1H, m), 6.88-6.97(2H, m), 7.03-7.08(1H, m), 7.26-7.43(10H, m). |
| 58 | 443 | 1.48-1.69(4H,m),1.84-1.97(2H,m), 2.02-2.16(2H,m) 2.20-2.38(4H, m), 2.42-2.53(2H, m), 2.80-3.08(5H, m), 6.52-6.63(3H, m), 7.05(1H, t, J=7.8Hz), 7.21-7.40(10H, m). |
| 59 | 443 | 1.62-1.73(4H, m), 1.92-2.07(2H, m) 2.08-2.20(2H, m), 2.20-2.38(4H, m),2.47-2.54(2H, m), 2.87-3.07(5H, m), 6.71(2H, d, J=8.8Hz), 6.98(2H, d, J=8.8Hz), 7.22-7.29(2H, m), 7.31-7.43(8H, m). |
| 60 | 441 | 1.62-1.84(4H, m), 1.97-2.20(4H, m), 2.26-2.42(3H, br), 2.27(3H, s), 2.46-2.68(3H, m), 2.84-3.09(5H, m), 7.02-7.16(3H, m), 7.18-7.22(1H, m), 7.24-7.46(10H, m). |
| 61 | 441 | 1.57-1.80(4H, m), 1.89-2.18(4H, m), 2.26-2.57(5H, m), 2.74-3.08(6H, m), 6.81-6.97(3H, m), 7.17-7.44(11H, m). |
| 62 | 441 | 1.50-1.85(8H, m), 1.95-2.47(5H, m), 2.29(3H, s), 2.48-2.63(2H, m), 2.90-3.15(4H, m), 7.08(4H, s), 7.20-7.30(2H, m), 7.30-7.45(8H, m). |
| 63 | 445 | 1.63-1.80(4H, m), 1.85-2.20(4H, m), 2.24-2.58(6H, m), 2.86-3.10(5H, m), 6.95-7.02(3H, m), 7.15(1H, t, J=7.3Hz), 7.24-7.45(10H, m). |
| 64 | 445 | 1.60-1.74(4H, m), 1.74-1.90(2H, m), 1.90-2.06(2H, m), 2.06-2.18(2H, m), 2.22-2.43(3H, m), 2.43-2.53(2H, m), 2.85-3.10(4H, m), 6.93(2H, t, J=8.8Hz), 7.11(1H, d, J=8.8Hz), 7.13(1H, d, J=8.8Hz), 7.20-7.30(2H, m), 7.30-7.43(8H,m). |
| 65 | 529 | 1.27(3H, t, J=7.3Hz), 1.64-1.85(4H, m), 2.00-2.23(4H, m), 2.27-2.42(3H, br), 2.46-2.60(2H, m), 2.86-3.09(6H, m), 4.23(2H, q, J=7.3Hz), 4.59(2H, s), 6.68(1H, d, J=7.8Hz), 6.93(1H, t, J=7.3Hz), 7.10(1H, t, J=7.8Hz), 7.20(1H, d, J=7.3Hz), 7.23-7.46(10H, m). |

**Table 16**

| | | |
|---|---|---|
| 66 | 529 | 1.55-1.85(5H, m), 1.90-2.20(3H, m), 2.06(3H, s), 2.25-2.40(3H, s), 2.45-2.60(2H, m), 2.80-3.10(6H, m), 4.13(2H, t, J=4.9Hz), 4.41(2H, t, J=4.9Hz), 6.79(1H, t, J=7.8Hz), 6.92(1H, t, J=7.3Hz), 7.13(1H, dt, J=7.8Hz, 1.5Hz), 7.18(1H, d, J=6.4Hz), 7.25-7.30(2H, m), 7.30-7.45(8H, m). |
| 67 | 529 | 1.64-1.80(4H, m), 1.94-2.17(5H, m), 2.08(3H, br), 2.23-2.55(6H, m), 2.84-3.09(4H, m), 4.13(2H, t, J=4.8Hz), 4.39(2H, t, J=4.8Hz), 6.69-6.82(3H, m), 7.17(1H, t, J=7.8Hz), 7.23-7.46(10H, m). |
| 68 | 529 | 1.62-1.77(4H, m), 1.77-2.03(4H, m), 2.03-2.17(2H, m), 2.08(3H, s), 2.23-2.42(3H, m), 2.43-2.53(2H, m), 2.87-3.07(4H, m), 4.12(2H, t, J=4.9Hz), 4.39(2H, t, J=4.9Hz), 6.81(2H, d, J=8.9Hz), 7.09 (2H, d, J=8.9Hz), 7.25-7.30(2H, m), 7.30-7.40(8H , m). |
| 69 | 487 | 1.60-1.75(2H, m), 1.85-2.00(2H, m), 2.00-2.10(2H, m), 2.33(3H, br.s), 2.40-2.50(2H, m), 2.65-2.75(1H, m), 2.85-3.15(7H, m), 3.39(1H, br.s), 3.90-4.00(2H, m), 4.02(2H, t, J=4.0Hz), 6.82(1H, d, J=9.0Hz), 6.88(1H, ddd, J= 8.3Hz, 8.3Hz, 1.0Hz), 7.10-7.20(1H, m), 7.20-7.50(11H, m). |
| 70 | 487 | 1.62-1.78(4H, m), 1.91-2.04(2H, m), 2.08-2.17(2H, m), 2.24-2.42(4H, m), 2.45-2.55(2H, m), 2.86-3.09(5H, m), 3.91(2H, t, J=4.5Hz), 4.03(2H, t, J=4.5Hz), 6.68-6.81(3H, m), 7.16(1H, t, J=7.8Hz), 7.23-7.46(10H, m). |
| 71 | 487 | 1.60-1.85(4H, m), 1.90-2.25(4H, m), 2.25-2.45(3H, m), 2.47-2.60(2H, m), 2.90-3.10(6H, m), 3.92(2H, t, J=4.4 Hz), 4.04(2H, t, J=4.4Hz), 6.82(2H, d, J=8.3Hz), 7.10 (2H, d, J=8.8Hz), 7.22-7.30(2H, m), 7.33-7.45(8H, m). |
| 72 | 501 | 1.72-2.03(4H, m), 2.28(3H, s), 2.35-2.69(7H, m), 2.95-3.08(1H, m), 2.97(3H, s), 3.21-3.40(1H, m), 4.40(2H, s), 6.63-6.95(4H, m), 7.24-7.42(10H, m). |
| 73 | 441 | 2.15-2.24(2H, m), 2.27-2.42(5H, m), 2.45-2.54(2H, m), 2.58-2.68(2H, m), 2.87-3.08(5H, m), 5.71(1H, s), 6.78-6.87(2H, m), 6.97-7.02(1H, m), 7.05-7.12(1H, m) 7.24-7.44(10H, m). |
| 74 | 527 | 1.26(3H, t, J=7.3Hz), 2.13-2.23(2H, m), 2.27-2.42(3H, br),2.47-3.12(5H, m), 4.22(2H, q, J=7.3Hz), 5.74(2H, s), 6.71(1H, d, J=8.3Hz), 6.92(1H, t, J=7.3Hz), 7.11-7.18(2H, m), 7.23-7.46(10H, m). |
| 75 | 485 | 2.31(3H, s), 2.70-2.84(4H, m), 2.89-3.04(2H, m), 3.01(3H, s), 3.07-3.20(2H, m), 3.49-3.68(2H, m), 3.88-4.03(4H, m), 5.56(1H, s), 6.80-6.92(2H, m), 7.05(1H, d, J=7.5Hz), 7.22(1H, t, J=7.5Hz), 7.27-7.46(10H, m). |

**Table 17**

| | | |
|---|---|---|
| 76 | 499 | 2.29(3H, s), 2.71-3.10(7H, m), 2.99(3H, s), 3.38-3.55(2H, m), 3.89-4.04(1H, m), 4.54(2H, m), 5.64(1H, s), 6.76(1H, d, J=7.8Hz), 6.93(1H, t, J=7.8Hz), 7.10-7.22(2H, m), 7.26-7.46(10H, m). |
| 77 | 467 | 1.47-1.58(2H, m), 1.62-1.72(2H, m), 2.21-2.30(2H, m), 2.33-2.57(4H, m), 2.60-2.68(2H, m), 3.04(2H, d, J=2.9Hz), 3.60(2H, t, J=7.0Hz), 5.70(1 H, s), 6.76-6.85(2H, m), 6.99(1H, d, J=7.3Hz), 7.08(1H, t, J=7.3Hz), 7.25-7.42(10H, m). |
| 78 | 469 | 1.46-1.57(2H, m), 1.60-1.82(6H, m), 2.00-2.26(4H, m), 2.42-2.59(4H, m), 2.77-3.02(2H, m), 3.54-3.65(2H, m), 6.67(1 H, d, J=7.8Hz), 6.76(1 H, t, J=7.3Hz), 6.96(1 H, t, J=7.8Hz), 7.06(1 H, d, J=7.3Hz), 7.20-7.38(10H, m). |
| 79 | 555 | 1.26(3H, dt, J=7.3, 2.2Hz), 1.47-1.58(2H, m), 1.61-1.85(6H, m), 2.03-2.32(4H, m), 2.42-2.63(4H, m), 2.92-3.10(3H, m), 3.52-3.66(2H, m), 2.4.23(2H, dq, J=7.3, 2.2Hz), 4.60(2H, s), 6.68(1 H, d, J=7.8Hz), 6.93(1 H, t, J=7.8Hz), 7.10(1H, t, J=7.8Hz), 7.20(1H, d, J=7.8Hz), 7.23-7.45(10H, m). |
| 80 | 555 | 1.48-1.58(2H, m), 1.61-1.85(6H, m), 2.06(3H, s), 2.08-2.37(4H, m), 2.42-2.65(4H, m), 2.85-3.14(3H, m), 3.54-3.63(2H, m), 4.08-4.15(2H, m), 4.36-4.44(2H, m), 6.79(1 H, d, J=7.5Hz), 6.92(1 H, t, J=7.5Hz), 7.09-7.21(2H, m), 7.23-7.42(10H, m). |
| 81 | 513 | 1.46-1.58(2H, m), 1.60-1.72(4H, m), 1.80-2.30(6H, m), 2.40-2.60(4H, m), 2.65-2.78(1H, m), 2.89-3.08(2H, m), 3.52-3.62(2H, m), 3.88-4.05(4H, m), 6.77-6.91(2H, m), 7.07-7.16(2H, m), 7.22-7.40(10H, m). |
| 82 | 527 | 1.47-1.58(2H, m), 1.61-1.72(2H, m), 1.75-2.06(6H, m), 2.32-2.53(4H, m), 2.57-2.73(3H, m), 2.97-3.10(1H, m), 3.26-3.42(1 H, m), 3.50-3.60(2H, m), 4.41(2H, s), 6.63-6.93(4H, m), 7.23-7.41(10H, m). |
| 83 | 557 | 1.20(3H,t,J=7.1Hz), 1.56(6H,s), 1.56-1.94(6H,m), 1.94-2.25(2H,m), 2.25-2.45(3H,br), 2.45-2.64(2H,br), 2.80-3.20(6H,m), 4.20(2H,q,J=7.1Hz), 6.61(1H,dd,J=7.8,1.0Hz), 6.90(1H,dt,J=7.8,1.0Hz), 7.01(1H,dt,J=7.8,1,0Hz), 7.17(1H,dd,J=7.8,1.0Hz), 7.25-7.32(2H,m), 7.32-7.47(8H,m). |
| 84 | 529 | 1.46(6H,s), 1.67-1.96(4H,m), 2.20-2.36(3H,br), 2.36-2.51(2H,m), 2.51-2.63(2H,m), 2.63-2.76(2H,m), 2.88-3.06(5H,m), 3.28-3.42(1H,m), 6.59-6.69(1H,m), 6.72-6.85(1H,m), 7.27-7.35(2H,m), 7.35-7.45(10H,m). |

### Test Example 1 (Effect confirmed with ileum specimens removed from guinea pigs)

Hartley male guinea pigs (body weight: about 500 g) were employed. The ileum (15-30 cm) was removed from the ileocecum of each guinea pig, to thereby prepare longitudinal muscle specimens having Auerbach's plexus. Opioid receptor activity of the test drugs was measured through a method proposed by Oka *et al.* (Eur. J. Pharmacol., 77; 137-141, 1982). Agonist activity is represented by the efficacy relative to the activity of DAMGO ([D-Ala², N-MePhe⁴, Gly⁵-ol]-enkephalin), which is a selective µ-opioid agonist. The fact that the test drugs are in fact opioid agonists had been confirmed through antagonism by naloxone.

The results are shown in the Table below.

**Table 18**

| Compound No. | Relative potency |
|---|---|
| 36 | 10 |
| 39 | 20 |
| 43 | 20 |
| 45 | 20 |
| 69 | 10 |
| DAMGO | 1.0 |
| Morphine | 0.14 |
| Loperamide | 4.3 |

### Test Example 2

Pain was induced in ICR male mice (5 weeks old) with fenvalerate for evaluation of analgesic effect of respective drugs. Specifically, fenvalerate (Na⁺ channel activator, 1 µg/site) was locally and subcutaneously administered to foot pads of the mice, and time during which the thus-induced pain response (licking or biting the foot pad) was observed was measured. The total length of time in which the pain response was manifested during 30 minutes following administration was employed as an index for evaluation of analgesic effect. Each of the test drugs had been locally administered to the same site 15 minutes before the administration of fenvalerate. Monohydrochloride of [2-[4-[4-(dimethylamino)-3,3-diphenyl-4-oxobutyl]piperazin-1-yl]phenoxyacetic acid (Example 45) was employed as a drug of the present invention. Loperamide and morphine, which are known to exhibit µ-opioid agonist activity, were employed as comparative drugs, and a solvent (10% DMSO) was employed as a control. The results (averaged on 10 mice per group) are shown in Fig. 1.

As is apparent from Fig. 1, the neuropathic pain relieving drug of the present invention was confirmed to exhibit statistically significant analgesic effect on pain response induced by fenvalerate.

### Industrial Applicability

2,2-Diphenylbutanamide derivatives of the present invention or salts thereof exhibit excellent µ-opioidagonism and analgesic activity against neuropathic pain, and thus are useful as peripheral analgesics, neuropathic pain relieving drugs, or like medicines.

## Claims

1. A 2,2-diphenylbutanamide derivative represented by the following formula (1): 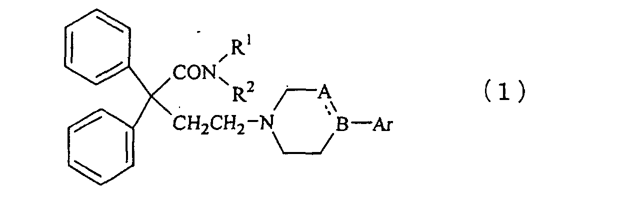 [wherein A represents -(CH₂)ₙ- (n is 1 or 2) or a methine (CH) group; when A is -CH₂-, B represents a methine group or a nitrogen atom, with A and B forming a single bond; when A is -(CH₂)₂-, B represents a nitrogen atom, with A and B forming a single bond; and when A is a methine group, B represents a quaternary carbon atom, with A and B forming a double bond;
each of R¹ and R², which are identical to or different from each other, represents a hydrogen atom, a lower alkyl group, or a cycloalkyl group, or R¹ and R² may form a heterocyclic ring together with the adjacent nitrogen atom; and Ar represents a phenyl group, a bicyclic aromatic ring, a monocyclic heterocyclic ring, a bicyclic heterocyclic ring, or a fluorene group, which may have a substituent represented by the following group: 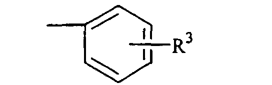 (wherein R³ represents a hydrogen atom, a halogen atom, a phenyl group, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; Y represents -COOR⁷, -OR⁸, -OCOR⁹, or-CONR¹⁰R¹¹ (wherein each of R⁷, R⁸, and R⁹ represents a hydrogen atom, a lower alkyl group, or a cycloalkyl group; and each of R¹⁰ and R¹¹ represents a hydrogen atom, a lower alkyl group, or a cycloalkyl group, or R¹⁰ and R¹¹ may form a heterocyclic ring together with the adjacent nitrogen atom); and m is 1 to 6)))]; or a salt of the derivative.

2. A 2,2-diphenylbutanamide derivative represented by the following formula (1-1) : 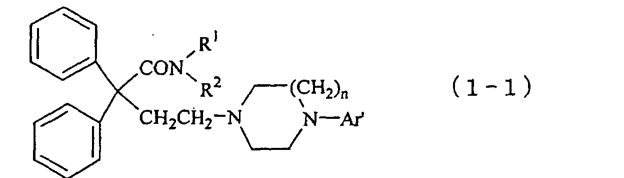 [wherein each of R¹ and R², which are identical to or different from each other, represents a hydrogen atom, a lower alkyl group, or a cycloalkyl group, or R¹ and R² may form a heterocyclic ring together with the adjacent nitrogen atom; n is 1 or 2; and Ar' represents a phenyl group, a bicyclic aromatic ring, a monocyclic heterocyclic ring, a bicyclic heterocyclic ring, or a fluorene group, which may have a substituent represented by the following group: 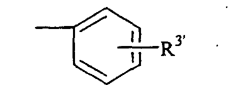 (wherein R^{3'} represents a hydrogen atom, a halogen atom, a phenyl group, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; Y represents -COOR⁷, -OR⁸, or -OCOR⁹ (wherein each of R⁷, R⁸, and R⁹ represents a hydrogen atom, a lower alkyl group, or a cycloalkyl group); and m is 1 to 6)))]; or a salt of the derivative.

3. A 2,2-diphenylbutanamide derivative represented by the following formula (1-2): 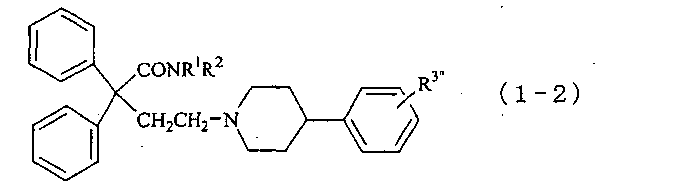 [wherein each of R¹ and R², which are identical to or different from each other, represents a hydrogen atom, a lower alkyl group, or a cycloalkyl group, or R¹ and R² may form a heterocyclic ring together with the adjacent nitrogen atom; and R³" represents a hydrogen atom, a halogen atom, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; Y represents -COOR⁷, -OR⁸, -OCOR⁹, or -CONR¹⁰R¹¹ (wherein each of R⁷, R⁸, and R⁹ represents a hydrogen atom, a lower alkyl group, or a cycloalkyl group; and each of R¹⁰ and R¹¹ represents a hydrogen atom, a lower alkyl group, or a cycloalkyl group, or R¹⁰ and R¹¹ may form a heterocyclic ring together with the adjacent nitrogen atom); and m is 1 to 6))]; or a salt of the derivative.

4. A 2,2-diphenylbutanamide derivative represented by the following formula (1-3): 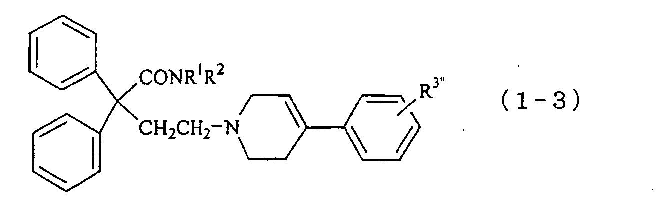 [wherein each of R¹ and R², which are identical to or different from each other, represents a hydrogen atom, a lower alkyl group, or a cycloalkyl group, or R¹ and R² may form a heterocyclic ring together with the adjacent nitrogen atom; and R³" represents a hydrogen atom, a halogen atom, a lower alkyl group, or -O-R⁴ (wherein R⁴ represents a hydrogen atom, a lower alkyl group, or -(CR⁵R⁶)ₘ-Y (wherein each of R⁵ and R⁶ represents a hydrogen atom or a lower alkyl group; Y represents -COOR⁷, -OR⁸, -OCOR⁹, or -CONR¹⁰R¹¹ (wherein each of R⁷, R⁸, and R⁹ represents a hydrogen atom, a lower alkyl group, or a cycloalkyl group; and each of R¹⁰ and R¹¹ represents a hydrogen atom, a lower alkyl group, or a cycloalkyl group, or R¹⁰ and R¹¹ may form a heterocyclic ring together with the adjacent nitrogen atom); and m is 1 to 6))]; or a salt of the derivative.

5. A medicine comprising, as an active ingredient, a 2,2-diphenylbutanamide derivative or a salt thereof as recited in any one of claims 1 through 4.

6. A medicine according to claim 5, which is a peripheral analgesic drug.

7. A medicine according to claim 5, which is a neuropathic pain relieving drug.

8. A medicinal composition comprising a 2,2-diphenylbutanamide derivative or a salt thereof as recited in any one of claims 1 through 4, and a pharmacologically acceptable carrier.

9. A method for treating peripheral pain or neuropathic pain, comprising administration of a 2,2-diphenylbutanamide derivative or a salt thereof as recited in any one of claims 1 through 4.

10. Use of a 2,2-diphenylbutanamide derivative or a salt thereof as recited in any one of claims 1 through 4 for producing a peripheral analgesic drug or a neuropathic pain relieving drug.
